# EUROPEAN PATENT APPLICATION

(11) **EP 4 821 935 A1**
(43) Date of publication of application: **14.10.2026**
(21) Application number: 24900025.8
(22) Date of filing: 09.12.2024
(51) Int. Cl.: C07D 513/22, A61K 31/5025, C07D 487/22, C07D 497/16, A61P 35/00

(54) **MACROCYCLIC COMPOUND CONTAINING AMIDE SUBSTITUTION**

(30) Priority: 08.12.2023 CN 202311693410; 05.12.2024 CN 202411783324
(71) Applicant: Chia Tai Tianqing Pharmaceutical Group Co., Ltd., Lianyungang, Jiangsu 222062 (CN)
(72) Inventor: ZHAO, Xiandong, Lianyungang, Jiangsu 222062 (CN); WANG, Bin, Lianyungang, Jiangsu 222062 (CN); MIN, Xiaolong, Lianyungang, Jiangsu 222062 (CN); WANG, Jinan, Lianyungang, Jiangsu 222062 (CN); ZHU, Lijuan, Lianyungang, Jiangsu 222062 (CN)
(74) Representative: Grünecker Patent- und Rechtsanwälte PartG mbB
(86) International application number: PCT/CN2024/137758
(87) International publication number: WO 2025/119392

(57) **Abstract**

The present application relates to the technical field of medicines, and relates to a macrocyclic compound containing amide substitution, in particular to a compound as shown in formula (I) or a pharmaceutically acceptable salt thereof, and a preparation method therefor, a pharmaceutical composition containing the compound, and a use thereof in the treatment of disease.

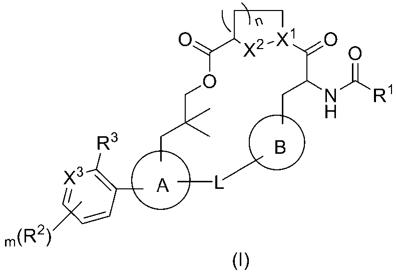

## Description

### CROSS-REFERENCE TO RELATED APPLICATIONS

The present application claims priority to and benefit of the Chinese Patent Application No. 202311693410.4 filed with China National Intellectual Property Administration on Dec. 08, 2023 and the Chinese Patent Application No. 202400000000.0 filed with China National Intellectual Property Administration on Dec. 05, 2024, the disclosure of each of which is incorporated herein by reference in its entirety.

### TECHNICAL FIELD

The present disclosure pertains to the technical field of medicines, and relates to a macrocyclic compound containing amide substitution, a stereoisomer thereof, or a pharmaceutically acceptable salt thereof, as well as a preparation method therefor, a pharmaceutical composition comprising the compound, and use thereof for treating diseases.

### BACKGROUND

The discovery of small-molecule inhibitors relies on the existence of suitable binding pockets on the protein surface. About 90% of proteins in the human body lack this feature, and such targets are generally considered undruggable; thus, innovative therapeutic targeting strategies are still required. It is reported that about 30% of human cancers are caused by mutations in Ras proteins (including K-Ras, H-Ras, and N-Ras). Ras proteins play a vital role in various human cancers, thereby becoming suitable targets in anti-cancer therapies. Specifically, dysregulation of Ras proteins caused by activating mutations, overexpression, or upstream activation can lead to the growth and proliferation of human tumor cells. For example, KRAS, as a small guanosine triphosphatase (GTPase), can cycle between an inactive [guanosine diphosphate (GDP)-bound, OFF] state and an active (GTP-bound, ON) state. KRAS in the active state can bind to and activate various effector proteins to regulate cell growth and proliferation. KRAS mutations stimulate excessive downstream signaling and proliferation, acting as critical oncogenic drivers closely related to the occurrence and development of various human cancers. Furthermore, mutations at codons 13 (e.g., G13D) and 61 (e.g., Q61K) in Ras also induce oncogenic activity in some cancers. Currently, literature has reported that drugs targeting RAS in the active state (ON) can inactivate oncogenic signals and lead to tumor regression in various human cancer models. However, no such drugs have been approved for marketing yet, and more efforts are still needed in the field to discover drugs against various cancers driven by Ras mutations.

### SUMMARY

The present disclosure relates to a compound of formula (I), a stereoisomer thereof, or a pharmaceutically acceptable salt thereof, 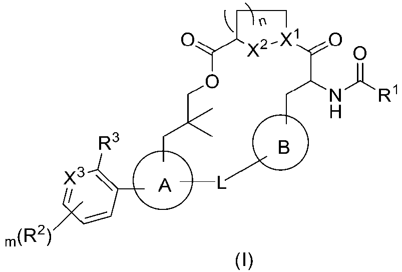 wherein
ring A is selected from the group consisting of C₆₋₁₀ aryl, 5- to 14-membered heteroaryl, and 5- to 14-membered heterocyclyl, wherein the C₆₋₁₀ aryl, 5- to 14-membered heteroaryl, or 5- to 14-membered heterocyclyl is optionally independently substituted with 1, 2, or 3 R^{a};
L is selected from the group consisting of -(CRR')_{q}-, -NH-(CRR')_{q}-, -O-(CRR')_{q}-, -S-(CRR')_{q}-, -(CRR')_{q}-(CH=CH)ᵢ-, -NH-(CH=CH)ᵢ-, -O-(CH=CH)ᵢ-, -S-(CH=CH)ᵢ-, -(CRR')_{q}-(C≡C)ᵢ-, -NH-(C≡C)ᵢ-, -O-(C≡C)ᵢ-, and -S-(C≡C)ᵢ-; R and R' are each independently selected from the group consisting of hydrogen, deuterium, halogen, -NH₂, -OH, C₁₋₆ alkyl, C₁₋₆ haloalkyl, C₁₋₆ heteroalkyl, C₃₋₆ cycloalkyl, and 3- to 6-membered heterocyclyl, wherein the C₃₋₆ cycloalkyl or 3- to 6-membered heterocyclyl is optionally independently substituted with one or more substituents selected from the group consisting of halogen, =O, -NH₂, -OH, -SH, and -CN; or R and R', together with the carbon atom to which they are attached, form C=O, C₃₋₆ cycloalkyl, or 3- to 6-membered heterocyclyl, wherein the C₃₋₆ cycloalkyl or 3- to 6-membered heterocyclyl is optionally independently substituted with one or more substituents selected from the group consisting of halogen, =O, -NH₂, -OH, -SH, and -CN;
ring B is selected from the group consisting of C₃₋₁₄ cycloalkyl, C₃₋₁₄ cycloalkenyl, 3- to 14-membered heterocyclyl, C₆₋₁₀ aryl, and 5- to 14-membered heteroaryl, wherein the C₃₋₁₄ cycloalkyl, C₃₋₁₄ cycloalkenyl, 3- to 14-membered heterocyclyl, C₆₋₁₀ aryl, or 5- to 14-membered heteroaryl is optionally independently substituted with 1, 2, or 3 R^{b}; R^{a} and R^{b} are each independently selected from the group consisting of halogen, =O, -NH₂, -OH, -SH, -CN, C₁₋₁₂ alkyl, C₁₋₁₂ heteroalkyl, C₂₋₁₂ alkenyl, C₂₋₁₂ alkynyl, C₃₋₈ cycloalkyl, 3- to 8-membered heterocyclyl, phenyl, and 5-to 6-membered heteroaryl, wherein the C₁₋₁₂ alkyl, C₁₋₁₂ heteroalkyl, C₂₋₁₂ alkenyl, or C₂₋₁₂ alkynyl is optionally independently substituted with one or more substituents selected from the group consisting of halogen, -NH₂, -OH, -SH, and -CN; the C₃₋₈ cycloalkyl, 3- to 8-membered heterocyclyl, phenyl, or 5- to 6-membered heteroaryl is optionally independently substituted with one or more substituents selected from the group consisting of halogen, =O, -NH₂, -OH, -SH, -CN, -COOH, C₁₋₆ alkyl, C₁₋₆ heteroalkyl, hydroxyl-substituted C₁₋₆ alkyl, C₁₋₆ haloalkyl, and amino-substituted C₁₋₆ alkyl;
R¹ is selected from the group consisting of C₁₋₆ alkyl, C₁₋₆ heteroalkyl, C₂₋₆ alkenyl, C₂₋₆ alkynyl, C₃₋₁₄ cycloalkyl, C₄₋₁₄ cycloalkenyl, 4- to 14-membered heterocyclyl, C₆₋₁₀ aryl, and 5- to 14-membered heteroaryl, wherein the C₁₋₆ alkyl, C₁₋₆ heteroalkyl, C₂₋₆ alkenyl, or C₂₋₆ alkynyl is optionally independently substituted with one or more substituents selected from the group consisting of halogen, -NH₂, -OH, -SH, -CN, and -COOH; the C₃₋₁₄ cycloalkyl, C₄₋₁₄ cycloalkenyl, 4- to 14-membered heterocyclyl, C₆₋₁₀ aryl, or 5- to 14-membered heteroaryl is optionally independently substituted with one or more substituents selected from the group consisting of halogen, =O, -NH₂, - OH, -SH, -CN, -COOH, C₁₋₆ alkyl, C₁₋₆ heteroalkyl, hydroxyl-substituted C₁₋₆ alkyl, C₁₋₆ haloalkyl, and amino-substituted C₁₋₆ alkyl;
each R² is independently selected from the group consisting of halogen, -NH₂, -OH, -SH, -CN, -C(O)NHC₁₋₆ alkyl, -C(O)N(C₁₋₆ alkyl)₂, -C(O)OC₁₋₆ alkyl, -OC(O)C₁₋₆ alkyl, -N(C₁₋₆ alkyl)C(O)C₁₋₆ alkyl, -NHS(O)₂C₁₋₆ alkyl, - S(O)₂NHC₁₋₆ alkyl, -S(O)₂N(C₁₋₆ alkyl)₂, -P(O)(C₁₋₆ alkyl)₂, C₁₋₆ alkyl, C₁₋₆ heteroalkyl, C₂₋₆ alkenyl, C₂₋₆ alkynyl, C₃₋₁₄ cycloalkyl, C₄₋₁₄ cycloalkenyl, 4- to 14-membered heterocyclyl, C₆₋₁₀ aryl, and 5- to 14-membered heteroaryl, wherein the C₁₋₆ alkyl, C₁₋₆ heteroalkyl, C₂₋₆ alkenyl, or C₂₋₆ alkynyl is optionally independently substituted with one or more substituents selected from the group consisting of halogen, -NH₂, -OH, -SH, -CN, and -COOH; the C₃₋₁₄ cycloalkyl, C₄₋₁₄ cycloalkenyl, 4- to 14-membered heterocyclyl, C₆₋₁₀ aryl, or 5- to 14-membered heteroaryl is optionally independently substituted with one or more substituents selected from the group consisting of halogen, =O, -NH₂, -OH, -SH, -CN, -COOH, C₁₋₆ alkyl, C₁₋₆ heteroalkyl, hydroxyl-substituted C₁₋₆ alkyl, C₁₋₆ haloalkyl, and amino-substituted C₁₋₆ alkyl;
X¹ and X³ are each independently selected from the group consisting of CH and N;
X² is selected from the group consisting of CH₂ and NH;
n is selected from the group consisting of 1, 2, 3, and 4;
m is selected from the group consisting of 0, 1, 2, 3, and 4;
i is selected from the group consisting of 1 and 2;
q is selected from the group consisting of 0, 1, 2, and 3;
R³ is selected from the group consisting of hydrogen, halogen, -NH₂, -OH, -SH, -CN, C₁₋₆ alkyl, and C₁₋₆ heteroalkyl, wherein the C₁₋₆ alkyl or C₁₋₆ heteroalkyl is optionally independently substituted with 1, 2, or 3 substituents selected from the group consisting of halogen, -NH₂, -OH, -SH, and -CN.

In some embodiments, the compound of formula (I) satisfies the following conditions:
when the structural fragment 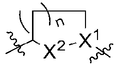 is selected from 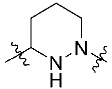 and ring A is selected from 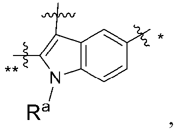 R¹ is not C₁₋₆ heteroalkyl or C₂₋₆ alkenyl optionally independently substituted with one or more substituents selected from the group consisting of halogen, -NH₂, -OH, -SH, -CN, and -COOH; and R¹ is not methyl or -CH₂CH(CH₃)₂; and R¹ is not C₃₋₁₄ cycloalkyl, 4- to 14-membered saturated heterocyclyl, pyridinyl, 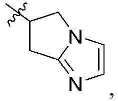 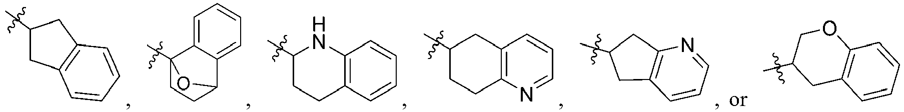 optionally independently substituted with one or more substituents selected from the group consisting of halogen, =O, -NH₂, - OH, -SH, -CN, -COOH, C₁₋₆ alkyl, C₁₋₆ heteroalkyl, hydroxyl-substituted C₁₋₆ alkyl, C₁₋₆ haloalkyl, and amino-substituted C₁₋₆ alkyl, wherein * indicates that a bond at the position is linked to L, and ** indicates that a bond at the position is linked to the structural fragment

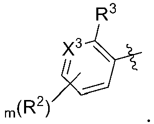

In some embodiments, the compound of formula (I) satisfies the following conditions:
when the structural fragment 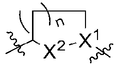 is selected from 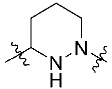 and ring A is selected from 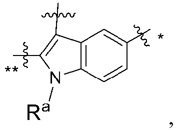 R¹ is not C₃₋₅ cycloalkyl or 6-membered heterocycloalkyl, wherein the C₃₋₅ cycloalkyl or 6-membered heterocycloalkyl is optionally substituted with 1 or 2 substituents selected from the group consisting of CN, C₁₋₃ alkyl, C₁₋₃ haloalkyl, and pyrimidinyl, wherein * indicates that a bond at the position is linked to L, and ** indicates that a bond at the position is linked to the structural fragment

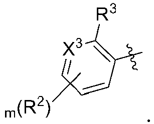

In some embodiments, ring A is selected from the group consisting of C₆₋₁₀ aryl, 5- to 10-membered heteroaryl, and 5- to 10-membered heterocyclyl, wherein the C₆₋₁₀ aryl, 5- to 10-membered heteroaryl, or 5- to 10-membered heterocyclyl is optionally independently substituted with 1, 2, or 3 R^{a}, wherein R^{a} is as defined in the present disclosure.

In some embodiments, ring A is selected from the group consisting of C₆₋₁₀ aryl and 5- to 10-membered heteroaryl, wherein the C₆₋₁₀ aryl or 5- to 10-membered heteroaryl is optionally independently substituted with 1 or 2 R^{a}, wherein R^{a} is as defined in the present disclosure.

In some embodiments, ring A is selected from the group consisting of phenyl, 5- to 6-membered heteroaryl, and 9-to 10-membered heteroaryl, wherein the phenyl, 5- to 6-membered heteroaryl, or 9- to 10-membered heteroaryl is optionally independently substituted with 1 or 2 R^{a}, wherein R^{a} is as defined in the present disclosure.

In some embodiments, ring A is selected from the group consisting of 5- to 6-membered heteroaryl and 9- to 10-membered heteroaryl, wherein the 5- to 6-membered heteroaryl or 9- to 10-membered heteroaryl is optionally independently substituted with 1 or 2 R^{a}, wherein R^{a} is as defined in the present disclosure.

In some embodiments, ring A is selected from 5-membered or 9-membered heteroaryl, wherein the 5-membered or 9-membered heteroaryl is optionally independently substituted with 1 or 2 R^{a}, wherein R^{a} is as defined in the present disclosure.

In some embodiments, ring A is selected from the group consisting of pyrrolyl, pyrazolyl, imidazolyl, oxazolyl, isoxazolyl, thiazolyl, isothiazolyl, triazolyl, furanyl, thienyl, pyridinyl, pyrazinyl, pyrimidinyl, pyridazinyl, indolyl, isoindolyl, benzofuranyl, benzothienyl, benzimidazolyl, and indazolyl, and ring A is optionally substituted with 1 or 2 R^{a}, wherein R^{a} is as defined in the present disclosure.

In some embodiments, ring A is selected from the group consisting of indolyl and pyrrolyl, wherein the indolyl or pyrrolyl is optionally independently substituted with 1 or 2 R^{a}, wherein R^{a} is as defined in the present disclosure.

In some embodiments, ring A is selected from the group consisting of phenyl and 5- to 6-membered or 9- to 10-membered heteroaryl, wherein the phenyl or 5- to 6-membered or 9- to 10-membered heteroaryl is optionally independently substituted with 1 or 2 substituents selected from the group consisting of halogen, C₁₋₄ alkyl, C₂₋₄ alkynyl, -NH₂, -OH, -SH, and -CN.

In some embodiments, ring A is selected from the group consisting of phenyl and 5- to 6-membered or 9- to 10-membered heteroaryl, wherein the phenyl or 5- to 6-membered or 9- to 10-membered heteroaryl is optionally independently substituted with 1 or 2 C₁₋₃ alkyl or C₂₋₄ alkynyl.

In some embodiments, ring A is selected from 5- to 6-membered or 9- to 10-membered heteroaryl, wherein the 5-to 6-membered or 9- to 10-membered heteroaryl is optionally independently substituted with 1 or 2 C₁₋₃ alkyl or C₂₋₄ alkynyl.

In some embodiments, ring A is selected from 5-membered or 9-membered heteroaryl, wherein the 5-membered or 9-membered heteroaryl is optionally independently substituted with 1 or 2 C₁₋₃ alkyl or C₂₋₄ alkynyl.

In some embodiments, ring A is selected from the group consisting of pyrrolyl, pyrazolyl, imidazolyl, oxazolyl, isoxazolyl, thiazolyl, isothiazolyl, triazolyl, furanyl, thienyl, pyridinyl, pyrazinyl, pyrimidinyl, pyridazinyl, indolyl, isoindolyl, benzofuranyl, benzothienyl, benzimidazolyl, and indazolyl, and ring A is optionally substituted with 1 or 2 C₁₋₃ alkyl or C₂₋₄ alkynyl.

In some embodiments, ring A is selected from the group consisting of indolyl and pyrrolyl, wherein the indolyl or pyrrolyl is substituted with 1 or 2 ethyl or ethynyl.

In some embodiments, ring A is selected from indolyl substituted with 1 or 2 ethyl or ethynyl.

In some embodiments, ring A is selected from the group consisting of 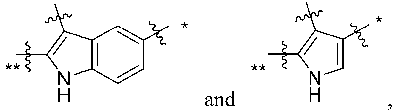 wherein * indicates that a bond at the position is linked to L, and ** indicates that a bond at the position is linked to the structural fragment 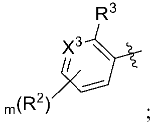 ring A is optionally independently substituted with 1 or 2 C₁₋₃ alkyl or C₂₋₄ alkynyl.

In some embodiments, ring A is selected from 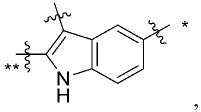 wherein * indicates that a bond at the position is linked to L, and ** indicates that a bond at the position is linked to the structural fragment 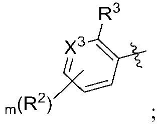 ring A is optionally independently substituted with 1 or 2 R^{a}.

In some embodiments, ring A is selected from the group consisting of 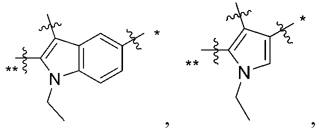 and 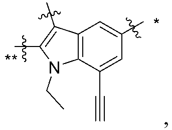 wherein * indicates that a bond at the position is linked to L, and ** indicates that a bond at the position is linked to the structural fragment

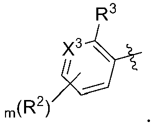

In some embodiments, ring A is selected from 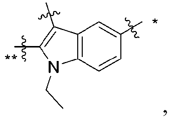 wherein * indicates that a bond at the position is linked to L, and ** indicates that a bond at the position is linked to the structural fragment

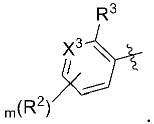

In some embodiments, L is selected from the group consisting of -(CRR')_{q}-, -(CRR')_{q}-(CH=CH)ᵢ-, and -(CRR')_{q}-(C≡C)ᵢ-, wherein R, R', q, and i are as defined in the present disclosure.

In some embodiments, L is selected from the group consisting of -(CRR')_{q}- and -(CRR')_{q}-(CH=CH)ᵢ-, wherein R, R', q, and i are as defined in the present disclosure.

In some embodiments, L is selected from the group consisting of a bond and -CH=CH-.

In some embodiments, L is a bond.

In some embodiments, L is -CH=CH-.

In some embodiments, R and R' are each independently selected from the group consisting of hydrogen, deuterium, halogen, -NH₂, -OH, C₁₋₃ alkyl, C₁₋₃ haloalkyl, and C₁₋₃ heteroalkyl. Alternatively, in some embodiments, R and R', together with the carbon atom to which they are attached, form C=O or cyclopropyl.

In some embodiments, R and R' are each independently selected from the group consisting of hydrogen, F, Cl, - NH₂, -OH, and methyl.

In some embodiments, R and R' are each independently hydrogen.

In some embodiments, ring B is selected from the group consisting of C₅₋₁₀ cycloalkyl, C₅₋₁₀ cycloalkenyl, 5- to 10-membered heterocyclyl, C₆₋₁₀ aryl, and 5- to 10-membered heteroaryl, wherein the C₅₋₁₀ cycloalkyl, C₅₋₁₀ cycloalkenyl, 5- to 10-membered heterocyclyl, C₆₋₁₀ aryl, or 5- to 10-membered heteroaryl is optionally substituted with 1, 2, or 3 R^{b}, wherein R^{b} is as defined in the present disclosure.

In some embodiments, ring B is selected from the group consisting of phenyl and 5- to 6-membered or 9- to 10-membered heteroaryl, wherein the phenyl or 5- to 6-membered or 9- to 10-membered heteroaryl is optionally independently substituted with 1 or 2 R^{b}, wherein R^{b} is as defined in the present disclosure.

In some embodiments, ring B is selected from the group consisting of phenyl and 5- to 6-membered heteroaryl, wherein the phenyl or 5- to 6-membered heteroaryl is optionally independently substituted with 1 or 2 R^{b}, wherein R^{b} is as defined in the present disclosure.

In some embodiments, ring B is selected from 5- to 6-membered heteroaryl, wherein the 5- to 6-membered heteroaryl is optionally substituted with 1 or 2 R^{b}, wherein R^{b} is as defined in the present disclosure.

In some embodiments, ring B is selected from the group consisting of pyrrolyl, pyrazolyl, imidazolyl, oxazolyl, isoxazolyl, thiazolyl, isothiazolyl, triazolyl, furanyl, thienyl, pyridinyl, pyrazinyl, pyrimidinyl, and pyridazinyl, and ring B is optionally substituted with 1 or 2 R^{b}, wherein R^{b} is as defined in the present disclosure.

In some embodiments, ring B is thiazolyl, wherein the thiazolyl is optionally substituted with 1 or 2 R^{b}, wherein R^{b} is as defined in the present disclosure.

In some embodiments, ring B is selected from the group consisting of C₅₋₁₀ cycloalkyl, C₅₋₁₀ cycloalkenyl, 5- to 10-membered heterocyclyl, C₆₋₁₀ aryl, and 5- to 10-membered heteroaryl, wherein the C₅₋₁₀ cycloalkyl, C₅₋₁₀ cycloalkenyl, 5- to 10-membered heterocyclyl, C₆₋₁₀ aryl, or 5- to 10-membered heteroaryl is optionally independently substituted with 1 or 2 substituents selected from the group consisting of -F, -Cl, -NH₂, -OH, -SH, and -CN.

In some embodiments, ring B is selected from the group consisting of phenyl, naphthyl, and 5- to 6-membered or 9- to 10-membered heteroaryl, wherein the phenyl, naphthyl, or 5- to 6-membered or 9- to 10-membered heteroaryl is optionally independently substituted with 1 or 2 substituents selected from the group consisting of -F, -Cl, -NH₂, -OH, -SH, and -CN.

In some embodiments, ring B is selected from the group consisting of phenyl and 5- to 6-membered heteroaryl, wherein the phenyl or 5- to 6-membered heteroaryl is optionally independently substituted with 1 or 2 substituents selected from the group consisting of -F, -Cl, -NH₂, -OH, -SH, and -CN.

In some embodiments, ring B is selected from the group consisting of phenyl and thiazolyl, wherein the phenyl is optionally substituted with 1 -OH.

In some embodiments, ring B is selected from the group consisting of phenyl and thiazolyl. In some embodiments, ring B is selected from 5- to 6-membered heteroaryl. In some embodiments, ring B is thiazolyl.

In some embodiments, ring B is 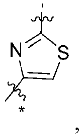 wherein * indicates that a bond at the position is linked to L, and ring B is optionally substituted with 1 or 2 substituents selected from the group consisting of -F, -Cl, -NH₂, -OH, -SH, and -CN.

In some embodiments, ring B is 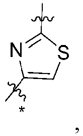 wherein * indicates that a bond at the position is linked to L.

In some embodiments, R^{a} and R^{b} are each independently selected from the group consisting of halogen, =O, -NH₂, -OH, -SH, -CN, C₁₋₈ alkyl, C₁₋₈ heteroalkyl, C₂₋₈ alkenyl, C₂₋₈ alkynyl, C₃₋₆ cycloalkyl, 3- to 6-membered heterocyclyl, phenyl, and 5- to 6-membered heteroaryl, wherein the C₁₋₈ alkyl, C₁₋₈ heteroalkyl, C₂₋₈ alkenyl, or C₂₋₈ alkynyl is optionally independently substituted with one or more substituents selected from the group consisting of halogen, -NH₂, -OH, -SH, and -CN; the C₃₋₆ cycloalkyl, 3- to 6-membered heterocyclyl, phenyl, or 5- to 6-membered heteroaryl is optionally independently substituted with one or more substituents selected from the group consisting of halogen, =O, -NH₂, -OH, -SH, -CN, -COOH, C₁₋₄ alkyl, C₁₋₄ heteroalkyl, hydroxyl-substituted C₁₋₄ alkyl, C₁₋₄ haloalkyl, and amino-substituted C₁₋₄ alkyl.

In some embodiments, R^{a} is selected from the group consisting of halogen, =O, -NH₂, -OH, -SH, -CN, C₁₋₃ alkyl, C₁₋₃ heteroalkyl, C₂₋₄ alkenyl, C₂₋₄ alkynyl, C₃₋₆ cycloalkyl, and 3- to 6-membered heterocyclyl, wherein the C₁₋₃ alkyl, C₁₋₃ heteroalkyl, C₂₋₄ alkenyl, or C₂₋₄ alkynyl is optionally independently substituted with one or more substituents selected from the group consisting of halogen, -NH₂, -OH, -SH, and -CN; the C₃₋₆ cycloalkyl or 3- to 6-membered heterocyclyl is optionally independently substituted with one or more substituents selected from the group consisting of halogen, =O, -NH₂, -OH, -SH, -CN, C₁₋₃ alkyl, C₁₋₃ heteroalkyl, and C₁₋₃ haloalkyl.

In some embodiments, R^{a} is selected from the group consisting of halogen, C₁₋₃ alkyl, -OC₁₋₃ alkyl, -NH(C₁₋₃ alkyl), -N(C₁₋₃ alkyl)₂, C₂₋₄ alkenyl, and C₂₋₄ alkynyl, wherein the C₁₋₃ alkyl, -OC₁₋₃ alkyl, -NH(C₁₋₃ alkyl), -N(C₁₋₃ alkyl)₂, C₂₋₄ alkenyl, or C₂₋₄ alkynyl is optionally independently substituted with one or more halogens.

In some embodiments, R^{a} is selected from the group consisting of F, Cl, C₁₋₃ alkyl, -OC₁₋₃ alkyl, -NH(C₁₋₂ alkyl), - N(C₁₋₂ alkyl)₂, and C₂₋₄ alkynyl, wherein the C₁₋₃ alkyl, -OC₁₋₃ alkyl, -NH(C₁₋₂ alkyl), -N(C₁₋₂ alkyl)₂, or C₂₋₄ alkynyl is optionally independently substituted with one or more halogens.

In some embodiments, R^{a} is selected from the group consisting of F, Cl, C₁₋₃ alkyl, -OC₁₋₃ alkyl, -NH(C₁₋₂ alkyl), - N(C₁₋₂ alkyl)₂, and C₂₋₄ alkynyl.

In some embodiments, R^{a} is selected from the group consisting of C₁₋₃ alkyl and C₂₋₄ alkynyl.

In some embodiments, R^{a} is selected from the group consisting of ethyl and ethynyl.

In some embodiments, R^{b} is selected from the group consisting of halogen, =O, -NH₂, -OH, -SH, -CN, C₁₋₆ alkyl, C₁₋₆ heteroalkyl, C₂₋₆ alkenyl, and C₂₋₆ alkynyl, wherein the C₁₋₆ alkyl, C₁₋₆ heteroalkyl, C₂₋₆ alkenyl, or C₂₋₆ alkynyl is optionally independently substituted with one or more substituents selected from the group consisting of halogen, -NH₂, -OH, -SH, and -CN.

In some embodiments, R^{b} is selected from the group consisting of halogen, =O, -NH₂, -OH, -SH, -CN, C₁₋₄ alkyl, and C₁₋₄ heteroalkyl, wherein the C₁₋₄ alkyl or C₁₋₄ heteroalkyl is optionally independently substituted with 1 or more substituents selected from the group consisting of F, Cl, -NH₂, -OH, -SH, and -CN.

In some embodiments, R^{b} is selected from the group consisting of halogen, =O, -NH₂, -OH, -SH, -CN, C₁₋₃ alkyl, - OC₁₋₃ alkyl, -NH(C₁₋₂ alkyl), and -N(C₁₋₂ alkyl)₂, wherein the C₁₋₃ alkyl, -OC₁₋₃ alkyl, -NH(C₁₋₂ alkyl), or -N(C₁₋₂ alkyl)₂ is optionally independently substituted with 1, 2, or 3 F atoms.

In some embodiments, R^{b} is selected from the group consisting of halogen, -NH₂, -OH, -SH, -CN, and C₁₋₃ alkyl.

In some embodiments, R^{b} is selected from the group consisting of F, Cl, and -OH.

In some embodiments, R¹ is selected from the group consisting of C₁₋₆ alkyl, C₁₋₆ heteroalkyl, C₂₋₆ alkenyl, C₂₋₆ alkynyl, C₃₋₁₀ cycloalkyl, C₅₋₁₀ cycloalkenyl, 4- to 10-membered heterocyclyl, C₆₋₁₀ aryl, and 5- to 10-membered heteroaryl, wherein the C₁₋₆ alkyl, C₁₋₆ heteroalkyl, C₂₋₆ alkenyl, or C₂₋₆ alkynyl is optionally independently substituted with one or more substituents selected from the group consisting of halogen, -NH₂, -OH, -SH, -CN, and -COOH; the C₃₋₁₀ cycloalkyl, C₅₋₁₀ cycloalkenyl, 4- to 10-membered heterocyclyl, C₆₋₁₀ aryl, or 5- to 10-membered heteroaryl is optionally independently substituted with one or more substituents selected from the group consisting of halogen, =O, -NH₂, -OH, -SH, -CN, -COOH, C₁₋₆ alkyl, C₁₋₆ heteroalkyl, and C₁₋₆ haloalkyl.

In some embodiments, R¹ is selected from the group consisting of C₁₋₄ alkyl, C₁₋₄ heteroalkyl, C₂₋₄ alkenyl, C₂₋₄ alkynyl, C₃₋₈ cycloalkyl, C₅₋₈ cycloalkenyl, and 4- to 8-membered heterocyclyl, wherein the C₁₋₄ alkyl, C₁₋₄ heteroalkyl, C₂₋₄ alkenyl, or C₂₋₄ alkynyl is optionally independently substituted with one or more substituents selected from the group consisting of halogen, -NH₂, -OH, -SH, -CN, and -COOH; the C₃₋₈ cycloalkyl, C₅₋₈ cycloalkenyl, or 4- to 8-membered heterocyclyl is optionally independently substituted with one or more substituents selected from the group consisting of halogen, =O, -NH₂, -OH, -SH, -CN, C₁₋₄ alkyl, C₁₋₄ haloalkyl, - OC₁₋₄ alkyl, -NH(C₁₋₃ alkyl), and -N(C₁₋₃ alkyl)₂.

In some embodiments, R¹ is selected from the group consisting of C₁₋₄ alkyl, C₁₋₄ heteroalkyl, C₃₋₆ cycloalkyl, C₅₋₈ cycloalkenyl, and 5- to 8-membered heterocyclyl, wherein the C₁₋₄ alkyl or C₁₋₄ heteroalkyl is optionally independently substituted with one or more substituents selected from the group consisting of halogen, -NH₂, -OH, -SH, and -CN; the C₃₋₆ cycloalkyl, C₅₋₈ cycloalkenyl, or 5- to 8-membered heterocyclyl is optionally independently substituted with one or more substituents selected from the group consisting of halogen, =O, -NH₂, -OH, -SH, -CN, C₁₋₃ alkyl, -OC₁₋₃ alkyl, C₁₋₃ haloalkyl, -NH(CH₃), and -N(CH₃)₂.

In some embodiments, R¹ is selected from the group consisting of C₁₋₄ haloalkyl, C₁₋₄ heteroalkyl, C₅₋₈ cycloalkenyl, and 5- to 8-membered partially unsaturated heterocyclyl, wherein the C₁₋₄ haloalkyl or C₁₋₄ heteroalkyl is optionally independently substituted with one or more substituents selected from the group consisting of halogen, -NH₂, -OH, -SH, and -CN; the C₅₋₈ cycloalkenyl or 5- to 8-membered partially unsaturated heterocyclyl is optionally independently substituted with one or more substituents selected from the group consisting of halogen, =O, -NH₂, - OH, -SH, -CN, C₁₋₃ alkyl, -OC₁₋₃ alkyl, C₁₋₃ haloalkyl, -NH(CH₃), and -N(CH₃)₂.

In some embodiments, R¹ is selected from the group consisting of C₅₋₈ cycloalkenyl and 5- to 8-membered partially unsaturated heterocyclyl, wherein the C₅₋₈ cycloalkenyl or 5- to 8-membered partially unsaturated heterocyclyl is optionally independently substituted with one or more substituents selected from the group consisting of halogen, =O, -NH₂, -OH, -SH, -CN, C₁₋₃ alkyl, -OC₁₋₃ alkyl, C₁₋₃ haloalkyl, -NH(CH₃), and -N(CH₃)₂.

In some embodiments, R¹ is selected from C₃₋₆ cycloalkyl, and n is 1, wherein the C₃₋₆ cycloalkyl is optionally substituted with one or more substituents selected from the group consisting of halogen, =O, -NH₂, -OH, -SH, -CN, C₁₋₃ alkyl, -OC₁₋₃ alkyl, C₁₋₃ haloalkyl, -NH(CH₃), and -N(CH₃)₂. In some embodiments, R¹ is selected from cyclopropyl, and n is 1, wherein the cyclopropyl is optionally substituted with one or more substituents selected from the group consisting of halogen, =O, -NH₂, -OH, -SH, -CN, C₁₋₃ alkyl, -OC₁₋₃ alkyl, C₁₋₃ haloalkyl, -NH(CH₃), and -N(CH₃)₂.

In some embodiments, R¹ is selected from C₃₋₆ cycloalkyl, and ring A is not 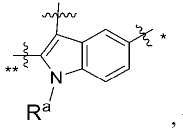 wherein the C₃₋₆ cycloalkyl is optionally substituted with one or more substituents selected from the group consisting of halogen, =O, -NH₂, -OH, -SH, -CN, C₁₋₃ alkyl, -OC₁₋₃ alkyl, C₁₋₃ haloalkyl, -NH(CH₃), and -N(CH₃)₂, wherein * indicates that a bond at the position is linked to L, and ** indicates that a bond at the position is linked to the structural fragment 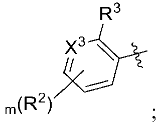 R^{a} is as defined in the present disclosure.

In some embodiments, R¹ is selected from cyclopropyl, and ring A is not 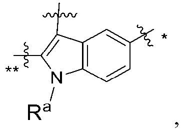 wherein the 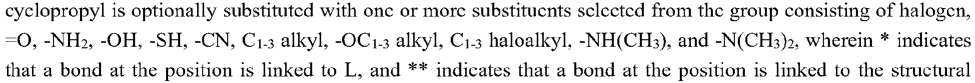 fragment 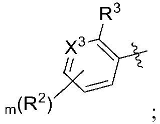 R^{a} is as defined in the present disclosure.

In some embodiments, R¹ is selected from the group consisting of C₁₋₃ haloalkyl, C₃₋₆ cycloalkyl, and C₅₋₇ cycloalkenyl, wherein the C₃₋₆ cycloalkyl or C₅₋₇ cycloalkenyl is optionally independently substituted with 1 or 2 substituents selected from the group consisting of halogen, -NH₂, C₁₋₃ alkyl, and C₁₋₃ haloalkyl.

In some embodiments, R¹ is selected from the group consisting of C₁₋₃ haloalkyl and C₅₋₇ cycloalkenyl, wherein the C₅₋₇ cycloalkenyl is optionally substituted with 1 or 2 substituents selected from the group consisting of halogen, - NH₂, C₁₋₃ alkyl, and C₁₋₃ haloalkyl.

In some embodiments, R¹ is selected from C₅₋₇ cycloalkenyl, wherein the C₅₋₇ cycloalkenyl is optionally substituted with 1 or 2 substituents selected from the group consisting of halogen, -NH₂, C₁₋₃ alkyl, and C₁₋₃ haloalkyl.

In some embodiments, R¹ is selected from the group consisting of halomethyl, cyclopropyl, cyclohexenyl, cyclopentenyl, and bicyclo[2.2.1]hept-2-enyl, wherein the cyclopropyl, cyclohexenyl, cyclopentenyl, or bicyclo[2.2.1]hept-2-enyl is optionally independently substituted with 1 or 2 F, Cl, -NH₂, CHF₂, or methyl.

In some embodiments, R¹ is selected from the group consisting of cyclohexenyl, cyclopentenyl, and bicyclo[2.2.1]hept-2-enyl, wherein the cyclohexenyl, cyclopentenyl, or bicyclo[2.2.1]hept-2-enyl is optionally independently substituted with 1 or 2 F, Cl, -NH₂, CHF₂, or methyl.

In some embodiments, R¹ is selected from cyclohexenyl, wherein the cyclohexenyl is optionally independently substituted with 1 or 2 F, Cl, -NH₂, CHF₂, or methyl.

In some embodiments, R¹ is selected from the group consisting of halomethyl, 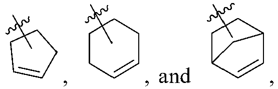 wherein the 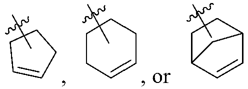 is optionally independently substituted with 1 or 2 substituents selected from the group consisting of halogen, -NH₂, C₁₋₃ alkyl, and C₁₋₃ haloalkyl.

In some embodiments, R¹ is selected from the group consisting of 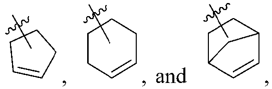 wherein the 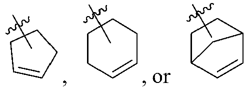 is optionally independently substituted with 1 or 2 substituents selected from the group consisting of halogen, -NH₂, C₁₋₃ alkyl, and C₁₋₃ haloalkyl.

In some embodiments, R¹ is selected from the group consisting of halomethyl and 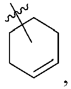 wherein the 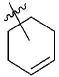 is optionally substituted with 1 or 2 substituents selected from the group consisting of halogen, -NH₂, C₁₋₃ alkyl, and C₁₋₃ haloalkyl. In some embodiments, R¹ is selected from 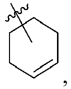 wherein the 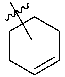 is optionally substituted with 1 or 2 substituents selected from the group consisting of halogen, -NH₂, C₁₋₃ alkyl, and C₁₋₃ haloalkyl.

In some embodiments, R¹ is selected from cyclopropyl, and n is 1, wherein the cyclopropyl is optionally substituted with one or more C₁₋₃ haloalkyl.

In some embodiments, R¹ is selected from cyclopropyl, and ring A is not 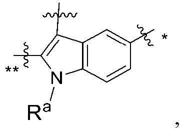 wherein the cyclopropyl is optionally substituted with one or more C₁₋₃ haloalkyl, wherein * indicates that a bond at the position is linked to L, and ** indicates that a bond at the position is linked to the structural fragment

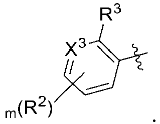

In some embodiments, R¹ is selected from C₁₋₃ haloalkyl.

In some embodiments, R¹ is selected from C₅₋₇ cycloalkenyl, wherein the C₅₋₇ cycloalkenyl is optionally substituted with 1 or 2 substituents selected from the group consisting of halogen, -NH₂, C₁₋₃ alkyl, C₁₋₃ haloalkyl, -OH, and CN.

In some embodiments, R¹ is selected from C₃₋₅ cycloalkyl, wherein the C₃₋₅ cycloalkyl is optionally substituted with 1 or 2 substituents selected from the group consisting of halogen, -NH₂, C₁₋₃ alkyl, C₁₋₃ haloalkyl, -OH, and CN.

In some embodiments, R¹ is selected from the group consisting of C₁₋₃ haloalkyl, C₅₋₇ cycloalkenyl, and C₃₋₅ cycloalkyl, wherein the C₅₋₇ cycloalkenyl or C₃₋₅ cycloalkyl is optionally substituted with 1 or 2 substituents selected from the group consisting of halogen, methyl, -NH₂, and -CHF₂.

In some embodiments, R¹ is selected from the group consisting of methyl, cyclohexenyl, cyclopropyl, cyclopentenyl, and bicyclo[2.2.1]hept-2-enyl, wherein the methyl is optionally substituted with 1 or 2 F or Cl, and the cyclohexenyl, cyclopropyl, cyclopentenyl, or bicycloheptenyl is optionally substituted with 1 or 2 methyl, -NH₂, or -CHF₂ substituents.

In some embodiments, R¹ is selected from the group consisting of

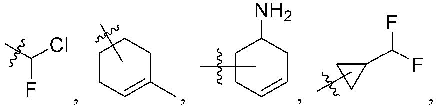

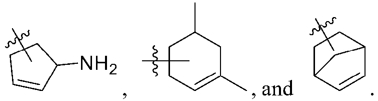

In some embodiments, R¹ is selected from the group consisting of

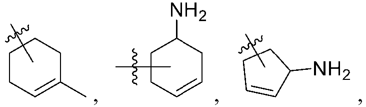

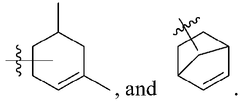

In some embodiments, R¹ is selected from the group consisting of

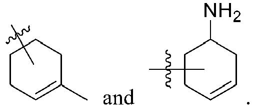

In some embodiments, R¹ is selected from the group consisting of

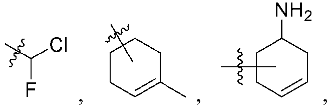

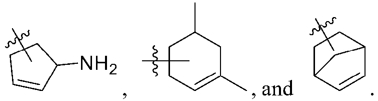

In some embodiments, R¹ is selected from the group consisting of

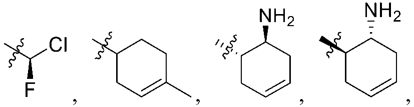

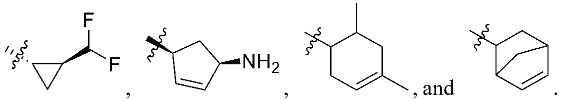

In some embodiments, R¹ is selected from the group consisting of

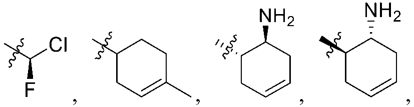

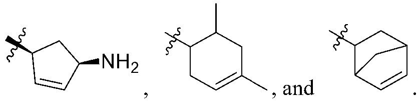

In some embodiments, R¹ is selected from 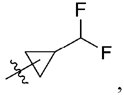 and n is 1.

In some embodiments, R¹ is selected from 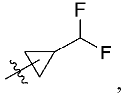 and ring A is not 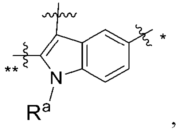 wherein * indicates that a bond at the position is linked to L, and ** indicates that a bond at the position is linked to the structural fragment

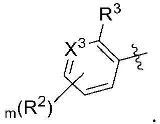

In some embodiments, X¹ is selected from N, X² is selected from the group consisting of CH₂ and NH, and X³ is selected from the group consisting of CH and N.

In some embodiments, X¹ and X³ are N, and X² is CH₂ or NH.

In some embodiments, X¹ and X³ are N, and X² is CH₂.

In some embodiments, X¹ and X³ are N, and X² is NH.

In some embodiments, n is selected from the group consisting of 1, 2, and 3.

In some embodiments, n is selected from the group consisting of 1 and 2.

In some embodiments, n is 1. In some embodiments, n is 2.

In some embodiments, the structural fragment 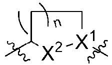 is selected from the group consisting of 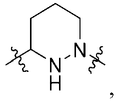 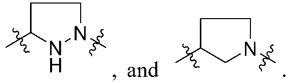 In some embodiments, the structural fragment 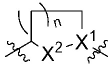 is selected from

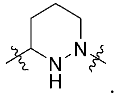

In some embodiments, the structural fragment 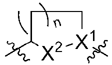 is selected from the group consisting of 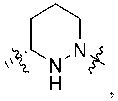 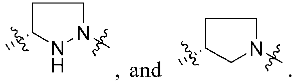 In some embodiments, the structural fragment 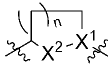 is selected from 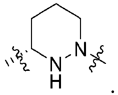 In some embodiments, each R² is independently selected from the group consisting of halogen, -NH₂, -OH, -SH, -CN, -C(O)NH(C₁₋₃ alkyl), -C(O)N(C₁₋₃ alkyl)₂, -C(O)OC₁₋₃ alkyl, -OC(O)C₁₋₃ alkyl, -N(C₁₋₃ alkyl)C(O)(C₁₋₃ alkyl), -NHS(O)₂(C₁₋₃ alkyl), -S(O)₂NH(C₁₋₃ alkyl), -S(O)₂N(C₁₋₃ alkyl)₂, -P(O)(C₁₋₃ alkyl)₂, C₁₋₆ alkyl, C₁₋₆ heteroalkyl, C₂₋₆ alkenyl, C₂₋₆ alkynyl, C₃₋₁₀ cycloalkyl, C₃₋₁₀ cycloalkenyl, 3- to 10-membered heterocyclyl, C₆₋₁₀ aryl, and 5- to 10-membered heteroaryl, wherein the C₁₋₆ alkyl, C₁₋₆ heteroalkyl, C₂₋₆ alkenyl, or C₂₋₆ alkynyl is optionally independently substituted with one or more substituents selected from the group consisting of halogen, -NH₂, -OH, -SH, -CN, and -COOH; the C₃₋₁₀ cycloalkyl, C₃₋₁₀ cycloalkenyl, 3- to 10-membered heterocyclyl, C₆₋₁₀ aryl, or 5- to 10-membered heteroaryl is optionally independently substituted with one or more substituents selected from the group consisting of halogen, =O, -NH₂, -OH, -SH, -CN, C₁₋₄ alkyl, C₁₋₄ heteroalkyl, and C₁₋₄ haloalkyl.

In some embodiments, each R² is independently selected from the group consisting of F, Cl, -NH₂, -OH, -SH, -CN, C₁₋₄ alkyl, C₁₋₄ heteroalkyl, C₃₋₈ cycloalkyl, C₅₋₈ cycloalkenyl, 5- to 10-membered heterocyclyl, C₆₋₁₀ aryl, and 5- to 6-membered heteroaryl, wherein the C₁₋₄ alkyl or C₁₋₄ heteroalkyl is optionally independently substituted with one or more substituents selected from the group consisting of halogen, -NH₂, -OH, -SH, -CN, and -COOH; the C₃₋₈ cycloalkyl, C₅₋₈ cycloalkenyl, 5- to 10-membered heterocyclyl, C₆₋₁₀ aryl, or 5- to 6-membered heteroaryl is optionally independently substituted with one or more halogens, -NH₂, -OH, -SH, -CN, C₁₋₃ alkyl, -OC₁₋₃ alkyl, C₁₋₃ haloalkyl, -NH(C₁₋₂ alkyl), or -N(C₁₋₂ alkyl)₂.

In some embodiments, each R² is independently selected from the group consisting of C₃₋₈ cycloalkyl, C₅₋₈ cycloalkenyl, and 5- to 8-membered heterocyclyl, wherein the C₃₋₈ cycloalkyl, C₅₋₈ cycloalkenyl, or 5- to 8-membered heterocyclyl is optionally independently substituted with one or more halogens, -NH₂, -OH, -SH, -CN, C₁₋₃ alkyl, -OC₁₋₃ alkyl, C₁₋₃ haloalkyl, -NH(CH₃), or -N(CH₃)₂.

In some embodiments, each R² is independently selected from 5- to 8-membered heterocyclyl, wherein the 5- to 8-membered heterocyclyl is optionally substituted with one or more substituents selected from the group consisting of halogen, -NH₂, -OH, -SH, -CN, C₁₋₃ alkyl, and C₁₋₃ haloalkyl.

In some embodiments, each R² is independently selected from 5- to 6-membered heterocyclyl containing 1 or 2 N or O atoms, wherein the 5- to 6-membered heterocyclyl is optionally substituted with one or more substituents selected from the group consisting of halogen, C₁₋₃ alkyl, and C₁₋₃ haloalkyl.

In some embodiments, each R² is independently selected from 5- to 6-membered partially unsaturated heterocyclyl containing 1 or 2 nitrogen atoms, wherein the 5- to 6-membered partially unsaturated heterocyclyl is optionally substituted with 1 or 2 substituents selected from the group consisting of F, Cl, and C₁₋₃ alkyl.

In some embodiments, each R² is independently selected from 5- to 8-membered heterocycloalkyl, wherein the 5-to 8-membered heterocycloalkyl is optionally substituted with one or more substituents selected from the group consisting of halogen, C₁₋₃ alkyl, and C₁₋₃ haloalkyl.

In some embodiments, each R² is independently selected from 5- to 6-membered heterocycloalkyl containing 1 or 2 heteroatoms selected from the group consisting of N and O, wherein the 5- to 6-membered heterocycloalkyl is optionally substituted with one or more substituents selected from the group consisting of halogen, C₁₋₃ alkyl, and C₁₋₃ haloalkyl.

In some embodiments, each R² is independently selected from 5- to 6-membered heterocycloalkyl containing 1 or 2 N atoms, wherein the 5- to 6-membered heterocycloalkyl is optionally substituted with 1 or 2 substituents selected from the group consisting of F, Cl, and C₁₋₃ alkyl.

In some embodiments, each R² is independently selected from piperazinyl, wherein the piperazinyl is optionally substituted with C₁₋₃ alkyl.

In some embodiments, each R² is independently selected from piperazinyl, wherein the piperazinyl is optionally substituted with methyl.

In some embodiments, each R² is independently selected from

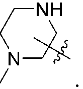

In some embodiments, each R² is independently

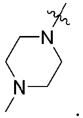

In some embodiments, m is selected from the group consisting of 0, 1, and 2.

In some embodiments, m is selected from the group consisting of 0 and 1.

In some embodiments, m is 0.

In some embodiments, m is 1.

In some embodiments, i is 1.

In some embodiments, q is selected from the group consisting of 0, 1, and 2.

In some embodiments, q is selected from the group consisting of 0 and 1. In some embodiments, q is 0.

In some embodiments, R³ is selected from the group consisting of hydrogen, halogen, -NH₂, -OH, -SH, -CN, C₁₋₄ alkyl, and C₁₋₄ heteroalkyl, wherein the C₁₋₄ alkyl or C₁₋₄ heteroalkyl is optionally independently substituted with 1, 2, or 3 substituents selected from the group consisting of halogen, -NH₂, -OH, -SH, and -CN.

In some embodiments, R³ is selected from the group consisting of F, Cl, -NH₂, -OH, -CN, C₁₋₃ alkyl, and C₁₋₃ heteroalkyl, wherein the C₁₋₃ alkyl or C₁₋₃ heteroalkyl is optionally independently substituted with 1 or 2 F atoms. In some embodiments, R³ is selected from C₁₋₃ heteroalkyl. In some embodiments, R³ is selected from C₃ heteroalkyl. In some embodiments, R³ is selected from C₁₋₃ alkoxy-substituted C₁₋₃ alkyl.

In some embodiments, R³ is 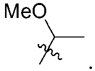 In some embodiments, R³ is

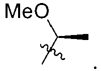

In some embodiments, the structural fragment 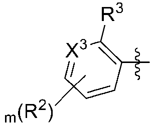 is selected from the group consisting of

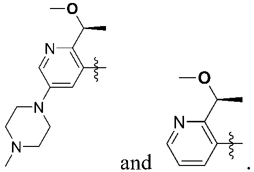

In some embodiments, the pharmaceutically acceptable salt of the compound of formula (I) described in the present disclosure is selected from a hydrochloride salt of the compound of formula (I).

In some embodiments, the compound of formula (I), the stereoisomer thereof, or the pharmaceutically acceptable salt thereof described in the present disclosure is selected from the group consisting of a compound of formula (I-1), a compound of formula (I-1A), a compound of formula (I-2), a compound of formula (I-2A), a compound of formula (I-3), a compound of formula (I-3A), a compound of formula (I-4), a compound of formula (I-4A), a compound of formula (I-5), and a compound of formula (I-5A), and stereoisomers thereof and pharmaceutically acceptable salts thereof, 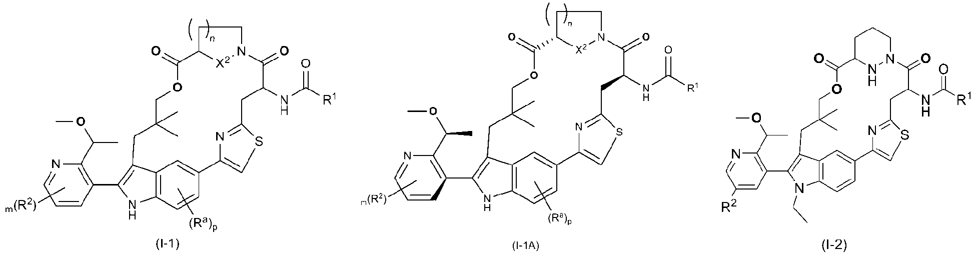 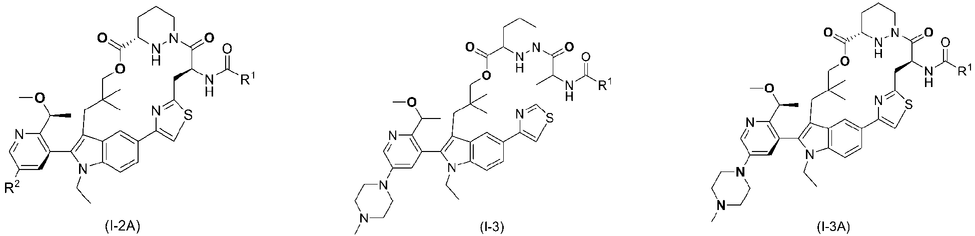 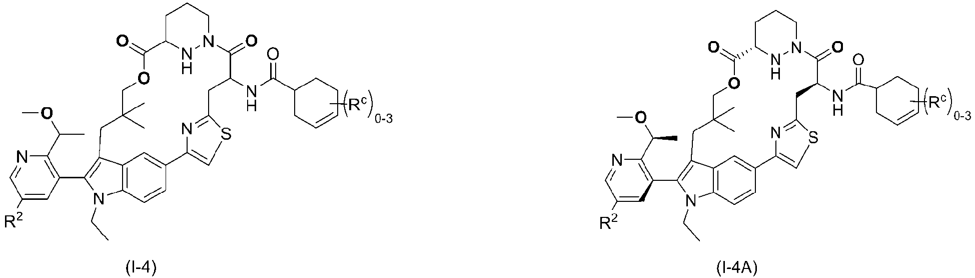 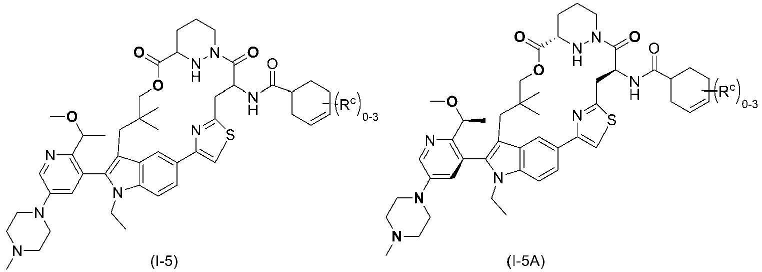
wherein p is selected from the group consisting of 1, 2, and 3;
X², R¹, R², R^{a}, m, and n are as defined in the present disclosure;
each R^{c} is independently selected from the group consisting of halogen, =O, -NH₂, -OH, -SH, -CN, -COOH, C₁₋₆ alkyl, C₁₋₆ heteroalkyl, hydroxyl-substituted C₁₋₆ alkyl, C₁₋₆ haloalkyl, and amino-substituted C₁₋₆ alkyl.

In some embodiments, each R^{c} is independently selected from the group consisting of -NH₂ and C₁₋₆ alkyl.

In some embodiments, each R^{c} is independently selected from the group consisting of -NH₂ and C₁₋₃ alkyl.

In some embodiments, each R^{c} is independently selected from the group consisting of -NH₂ and methyl.

It should be understood that any embodiment of the compounds of the present disclosure as described above, and any specific substituent set forth herein with respect to particular X¹, X², X³, ring A, L, ring B, R¹, R², R³, R^{a}, R^{b}, R^{c}, R, and R' in the compounds of the present disclosure as described above, may be independently combined with other embodiments and/or substituents of the compounds of the present disclosure to form embodiments of the present disclosure not specifically set forth above. Furthermore, where a range of substituents is disclosed in specific embodiments and/or claims with respect to any particular X¹, X², X³, ring A, L, ring B, R¹, R², R³, R^{a}, R^{b}, R^{c}, R, and R' substituents, it should be understood that one or more substituents may be deleted from the range and the remaining range of substituents should also be considered an embodiment of the present disclosure.

The present disclosure provides compounds as follows, stereoisomers thereof, or pharmaceutically acceptable salts thereof,

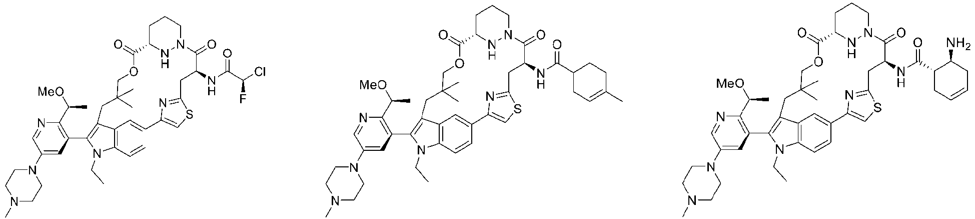

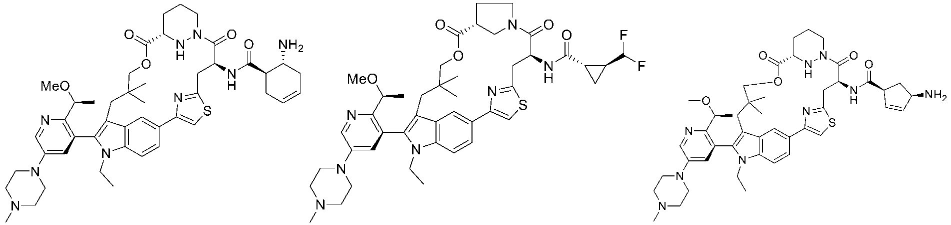

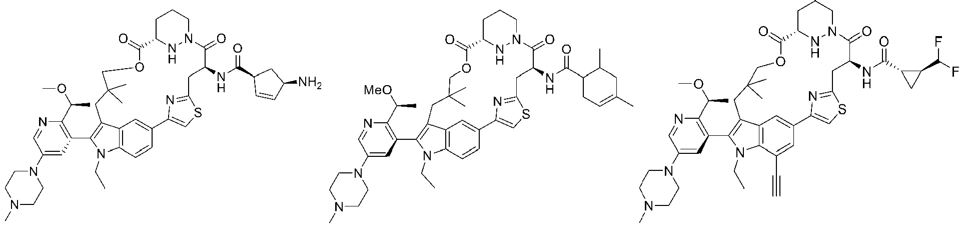

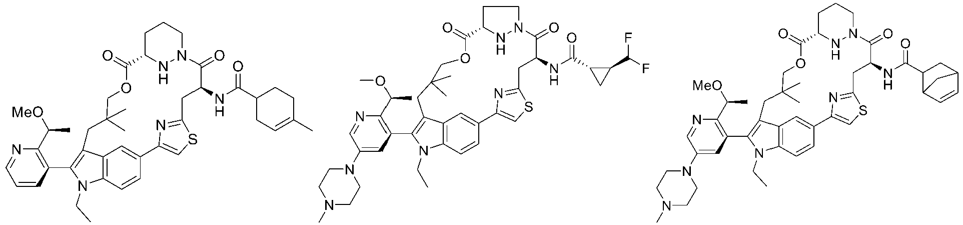

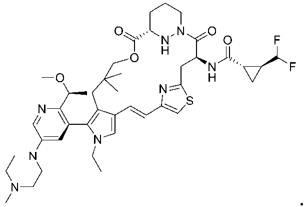

In another aspect, the present disclosure provides a pharmaceutical composition, which comprises the compound, the stereoisomer thereof, or the pharmaceutically acceptable salt thereof described in the present disclosure above. In some embodiments, the pharmaceutical composition of the present disclosure further comprises a pharmaceutically acceptable excipient.

In another aspect, the present disclosure provides a method for treating a Ras protein-related disease in a mammal, which comprises administering to a mammal, preferably a human, in need of such treatment a therapeutically effective amount of the compound, the stereoisomer thereof, or the pharmaceutically acceptable salt thereof, or the pharmaceutical composition thereof described above.

In another aspect, the present disclosure provides use of the compound, the stereoisomer thereof, or the pharmaceutically acceptable salt thereof, or the pharmaceutical composition thereof described above for preparing a medicament for treating a Ras protein-related disease.

In another aspect, the present disclosure provides use of the compound, the stereoisomer thereof, or the pharmaceutically acceptable salt thereof, or the pharmaceutical composition thereof described above for treating a Ras protein-related disease.

In another aspect, the present disclosure provides the compound, the stereoisomer thereof, or the pharmaceutically acceptable salt thereof, or the pharmaceutical composition thereof described above for treating a Ras protein-related disease. In some embodiments, the Ras protein-related disease is selected from cancer.

### Technical Effects

The compounds of the present disclosure exhibit relatively good inhibitory activity against the proliferation of AsPc-1 cells and Capan-1 cells, metabolic stability in liver microsomes *in vitro,* and good *in vivo* pharmacodynamic and pharmacokinetic properties.

### Definitions

Unless otherwise stated, the following terms used in the present disclosure shall have the following meanings. A certain term, unless otherwise specifically defined, should not be considered uncertain or unclear, but interpreted according to its common meaning in the art. When referring to a trade name, it is intended to refer to its corresponding commercial product or its active ingredient.

The term "substituted" means that any one or more hydrogen atoms on a specific atom are substituted with a substituent, as long as the valence of the specific atom is normal and the resulting compound is stable. When the substituent is oxo (i.e., =O), it means that two hydrogen atoms are substituted; oxo substitutions do not occur on aromatic groups.

The term "optional" or "optionally" means that the subsequently described event or circumstance may or may not occur, and the description includes instances where the event or circumstance occurs and instances where it does not. For example, ethyl being "optionally" substituted with halogen means that the ethyl may be unsubstituted (-CH₂CH₃), monosubstituted (e.g., -CH₂CH₂F), polysubstituted (e.g., -CHFCH₂F, -CH₂CHF₂, etc.), or fully substituted (-CF₂CF₃). It will be appreciated by those skilled in the art that for any group comprising one or more substituents, no substitution or substitution pattern that is sterically impossible and/or cannot be synthesized is introduced.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms; "C₁₋₃" means that the group may have 1 carbon atom, 2 carbon atoms, or 3 carbon atoms. "One or more" used herein refers to an integer ranging from one to ten. For example, "one or more" refers to one, two, three, four, five, six, seven, eight, nine, or ten; or "one or more" refers to one, two, three, four, five, or six; or "one or more" refers to one, two, or three.

When any variable (e.g., R) occurs more than once in the constitution or structure of a compound, the definition of the variable in each case is independent. Therefore, for example, if a group is substituted with 2 R, the definition of each R is independent.

When a bond of a substituent is cross-linked to two atoms on a ring, the substituent can be bonded to any atom on the ring. For example, the structural unit 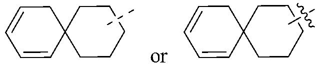 represents that substitution may occur at any one position of cyclohexyl or cyclohexadienyl.

The groups or structural moieties in the present application, such as -NH-(CRR')_{q}-, -O-(CRR')_{q}-, -S-(CRR')_{q}-, - (CRR')_{q}-(CH=CH)ᵢ-, -NH-(CH=CH)ᵢ-, -O-(CH=CH)ᵢ-, -S-(CH=CH)ᵢ-, -(CRR')_{q}-(C≡C)ᵢ-, -NH-(C≡C)ᵢ-, -O-(C≡C)ᵢ-, or -S-(C≡C)ᵢ-, and specific options thereof, may optionally follow a left-to-right reading order, so as to be correspondingly linked to the group or the left-side group and right-side group of the moiety in the general formula, respectively; specifically, as in -(CRR')_{q}-(CH=CH)ᵢ-, according to the left-to-right reading order, -(CRR')_{q}- is linked to the left-side ring A in the general formula, and -(CH=CH)ᵢ- is linked to the right-side ring B. Optionally, for example, the groups or structural moieties described above follow a right-to-left reading order; specifically, as in - (CRR')_{q}-(CH=CH)ᵢ-, according to the right-to-left reading order, -(CH=CH)ᵢ- is linked to the left-side ring A, and - (CRR')_{q}- is linked to the right-side ring B in the general formula.

The term "halo" or "halogen" refers to fluorine, chlorine, bromine, and iodine.

The term "hydroxyl" refers to an -OH group.

The term "amino" refers to an -NH₂ group.

The term "nitro" refers to an -NO₂ group.

The term "cyano" refers to a -CN group.

The term "alkyl" refers to hydrocarbyl with a general formula of CₙH₂ₙ₊₁. The alkyl may be linear or branched. For example, the term "C₁₋₆ alkyl" refers to alkyl containing 1 to 6 carbon atoms (e.g., methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, etc.). Similarly, the alkyl moieties (i.e., alkyl) of alkoxy, alkylamino, dialkylamino, alkylsulfonyl, and alkylthio have the same definition as described above.

The term "heteroalkyl" refers to linear or branched alkyl consisting of a certain number of carbon atoms and at least one skeleton chain heteroatom or heteroatom group; it has preferably 1 to 14 carbons, more preferably 1 to 10 carbons, even more preferably 1 to 6 carbons, and most preferably 1 to 3 carbons in the chain. The heteroatom is preferably selected from the group consisting of S, O, and N heteroatoms, and the number of the heteroatoms is preferably selected from the group consisting of 1, 2, and 3, wherein the nitrogen and sulfur atoms are optionally oxidized, and the nitrogen atom is optionally quaternized. The heteroatom group is preferably selected from the group consisting of -C(=O)O-, -C(=O)-, -C(=S)-, -S(=O), -S(=O)₂-, -C(=O)N(H)-, -N(H)-, -C(=NH)-, -S(=O)₂N(H)-, and -S(=O)N(H)-. A numerical range (e.g., C₁₋₆ heteroalkyl) refers to the number of carbons in the chain, which in this example refers to the inclusion of 1-6 carbon atoms. For example, a -CH₂OCH₂CH₃ group is referred to as C₃ heteroalkyl. The heteroatom or heteroatom group can be located at any interior position of the heteroalkyl, including the position at which the alkyl is attached to the rest of the molecule. In the heteroalkyl, the moiety directly linked to the parent structure may optionally be a carbon atom, a heteroatom, or a heteroatom group. Exemplary heteroalkyl includes alkyl ether, secondary alkylamine and tertiary alkylamine, amide, alkyl sulfide, tertiary aminoalkyl or secondary aminoalkyl, and the like, including alkoxy, alkylthio, and alkylamino; unless otherwise specified, C₁₋₆ heteroalkyl includes C₁, C₂, C₃, C₄, C₅, and C₆ heteroalkyl, e.g., C₁₋₆ alkoxy, C₁₋₆ alkylthio, or C₁₋₆ alkylamino.

The term "alkoxy" refers to -O-alkyl.

The term "bicyclic" refers to a cyclic group containing two rings, which may be fully saturated, partially saturated, or aromatic. The bicyclic ring may consist entirely of C atoms or may contain one or more heteroatoms, for example, selected from the group consisting of N, O, S, and P. The bicyclic ring may be a fused ring, a bridged ring, or a spiro ring.

The term "cycloalkyl" refers to a fully saturated carbocyclic ring. Unless otherwise specified, the carbocyclic ring is usually a 3- to 10-membered ring. Unless otherwise specified, the cycloalkyl may be monocyclic, bicyclic, or tricyclic. Non-limiting examples of the cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like.

The term "heterocyclyl" refers to a non-aromatic ring that is fully saturated or partially unsaturated (but not a fully unsaturated heteroaromatic group) and may exist as a monocyclic ring, a bridged ring, a fused ring, or a spiro ring. Unless otherwise specified, the heterocyclic ring is usually a 3- to 20-membered ring, a 3- to 15-membered ring, a 3- to 12-membered ring, a 3- to 10-membered ring (e.g., a 3-membered, 4-membered, 5-membered, 6-membered, 7-membered, 8-membered, 9-membered, or 10-membered ring), a 4- to 8-membered ring, a 5- to 8-membered ring, or a 5- to 6-membered ring containing 1 to 3 heteroatoms (preferably 1 or 2 heteroatoms) independently selected from the group consisting of sulfur, oxygen, nitrogen, phosphorus, silicon, and/or boron. Non-limiting examples of the heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4H-pyranyl, morpholinyl, thiomorpholinyl, tetrahydrothienyl, and the like.

The term "cycloalkenyl" refers to a non-aromatic carbocyclic ring that is not fully saturated and may exist as a monocyclic ring, a bicyclic bridged ring, or a spiro ring. Unless otherwise specified, the carbocyclic ring is usually a 4- to 16-membered ring, a 4- to 12-membered ring, a 4- to 10-membered ring, or a 4- to 8-membered ring (specifically, such as a 5-membered, 6-membered, 7-membered, 8-membered, 9-membered, 10-membered, or 11-membered ring). Non-limiting examples of the cycloalkenyl include, but are not limited to, cyclopentenyl, cyclopentadienyl, cyclohexenyl, cyclohexadienyl, cycloheptenyl, cycloheptadienyl, and the like.

The term "heterocycloalkenyl" includes cycloalkenyl in which one or more carbon atoms are substituted with a heteroatom, specifically, for example, cycloalkenyl in which up to 3 carbon atoms, up to 2 carbon atoms, or in one embodiment, 1 carbon atom, are each independently replaced by N, O, or S(O), provided that at least one cycloalkenyl carbon-carbon double bond is preserved. The heterocycloalkenyl may be a cyclic group that exists as a monocyclic ring, a bridged ring, or a spiro ring and may be a 3- to 16-membered ring (e.g., a 3- to 12-membered or 5- to 8-membered ring, specifically, such as a 5-membered, 6-membered, 7-membered, 8-membered, 9-membered, 10-membered, or 11-membered ring). Examples of the heterocycloalkenyl include, but are not limited to, dihydropyridinyl, dihydropyrrolyl, tetrahydropyridinyl, tetrahydroazepinyl, or azaspirooctenyl.

The term "aryl" refers to an all-carbon aromatic monocyclic or fused polycyclic group having a conjugated π-electron system. Unless otherwise specified, the aryl may have 6-20 carbon atoms, 6-14 carbon atoms, 6-12 carbon atoms, or 6-10 carbon atoms. Non-limiting examples of the aryl include, but are not limited to, phenyl, naphthyl, anthryl, 1,2,3,4-tetrahydronaphthalene, and the like, and preferably, the aryl is phenyl. In some embodiments, the aryl may be phenyl fused with heterocyclyl or cycloalkyl, forming 2 or more rings (e.g., 2, 3, 4 rings, etc.), and the ring in the aryl that is directly linked to the parent structure is a benzene ring; non-limiting examples include, but are not limited to, 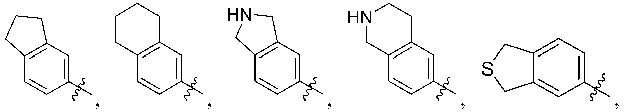 or the like.

The term "heteroaryl" refers to a monocyclic or polycyclic system that contains at least one ring atom selected from the group consisting of N, O, S(O)ₙ, and P(O)ₙ (wherein n is 0, 1, or 2), with the remaining ring atoms being C, and that has at least one aromatic ring that is a heteroaromatic ring. Unless otherwise specified, the heteroaryl may be monocyclic, bicyclic, or tricyclic. Unless otherwise specified, the heteroaryl may have a single 5- to 8-membered ring, or multiple fused rings containing 6 to 14, especially 6 to 10 ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like. In some embodiments, the heteroaryl may be 6-membered heteroaryl fused with heterocyclyl or cycloalkyl, forming 2 or more rings (e.g., 2, 3, 4 rings, etc.), and the ring in the heteroaryl that is directly linked to the parent structure is a heteroaromatic ring; non-limiting examples include, but are not limited to, 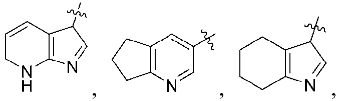 or the like.

Unless otherwise specified, the term "hetero" refers to a heteroatom or a heteroatom group (i.e., a heteroatom-containing group), including atoms other than carbon (C) and hydrogen (H) and groups containing such heteroatoms; for example, heteroatoms include, but are not limited to, oxygen (O), nitrogen (N), sulfur (S), phosphorus (P), silicon (Si), germanium (Ge), aluminum (Al), and boron (B); specific heteroatoms or heteroatom groups are, for example, -O-, -S-, -N=, =O, =S, -P(=O)-, -P(=O)₂-, -P(=O)O-, -P(=O)₂O-, -C(=O)O-, -C(=O)-, -C(=S)-, -S(=O), -S(=O)₂-, and optionally substituted -C(=O)N(H)-, -N(H)-, -C(=NH)-, -S(=O)₂N(H)-, or -S(=O)N(H)-. Preferably, the term "hetero" means that a heteroatom or a heteroatom of a heteroatom group (i.e., a heteroatom-containing group) is selected from the group consisting of oxygen, nitrogen, and sulfur.

The term "treat" or "treatment" means administering the compound or formulation described in the present disclosure to ameliorate or eliminate a disease or one or more symptoms associated with the disease, including:
(i) inhibiting a disease or disease state, i.e., arresting its progression; and
(ii) alleviating a disease or disease state, i.e., causing the regression of the disease or disease state.

The term "therapeutically effective amount" refers to an amount of the compound of the present disclosure for (i) treating the specific disease, condition, or disorder described herein; (ii) alleviating, ameliorating, or eliminating one or more symptoms of the specific disease, condition, or disorder described herein, or (iii) preventing or delaying onset of one or more symptoms of the specific disease, condition, or disorder described herein. The amount of the compound of the present disclosure constituting the "therapeutically effective amount" varies depending on the compound, the disease state and its severity, the mode of administration, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds, the stereoisomers thereof, or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, such as carbohydrates, wax, water-soluble and/or water-swellable polymers, hydrophilic or hydrophobic materials, gelatin, oil, solvents, water, and the like.

The word "comprise" and variations thereof such as "comprises" or "comprising" should be understood in an open, non-exclusive sense, i.e., "including but not limited to".

Unless otherwise specified, terms in the singular form shall be deemed to include the plural form and vice versa. Unless otherwise specified, the word "a" or "an" refers to "at least one". Unless otherwise stated, "or" is used to mean "and/or".

The compounds of the present disclosure may have particular geometric or stereoisomeric forms. All such compounds are contemplated in the present disclosure, including *cis* and *trans* isomers, (-)- and (+)-enantiomers, (*R*)- and (*S*)-enantiomers, diastereoisomers, (*D*)-isomers, (*L*)-isomers, and racemic mixtures and other mixtures thereof, such as enantiomerically or diastereoisomerically enriched mixtures, all of which are encompassed within the scope of the present disclosure. Additional asymmetric carbon atoms may be present in substituents such as alkyl. All such isomers and mixtures thereof are encompassed within the scope of the present disclosure.

Unless otherwise stated, "(*D*)" or "(+)" stands for dextrorotation, "(*L*)" or "(-)" stands for levorotation, and "(*DL*)" or "(+)" stands for racemization.

Unless otherwise stated, the absolute configuration of a stereogenic center is represented by a wedged solid bond ( 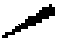 ) and a wedged dashed bond ( 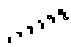 ), and the relative configuration of a stereogenic center is represented by a straight solid bond ( 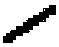 ) and a straight dashed bond ( 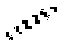 ).

Optically active (*R*)- and (*S*)-isomers and *D* and *L* isomers can be prepared by chiral synthesis, chiral reagents, or other conventional techniques. An enantiomer of a certain compound of the present disclosure can be prepared by asymmetric synthesis or derivatization using a chiral additive, wherein the resulting diastereoisomeric mixture is separated and the auxiliary group is cleaved so as to provide the desired pure enantiomer. Alternatively, when the molecule comprises a basic functional group (e.g., amino) or an acidic functional group (e.g., carboxyl), the compound reacts with an appropriate optically active acid or base to form a salt of the diastereoisomer, which is then subjected to diastereoisomeric resolution through conventional methods well known in the art to give the pure enantiomer. Furthermore, the enantiomer and the diastereoisomer are generally separated through chromatography using a chiral stationary phase, optionally in combination with chemical derivatization (e.g., carbamate generated from amines).

The present disclosure also includes isotopically labeled compounds of the present disclosure, which are identical to those recited herein but have one or more atoms replaced by an atom having an atomic mass or mass number different from the atomic mass or mass number generally found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, ³⁶Cl, and the like.

Certain isotopically labeled compounds of the present disclosure (e.g., those labeled with ³H and ¹⁴C) can be used to analyze compounds and/or substrate tissue distribution. Tritium (i.e., ³H) and carbon-14 (i.e., ¹⁴C) isotopes are particularly preferred for their ease of preparation and detectability. Positron emitting isotopes, such as ¹⁵P, ¹³N, ¹¹C, and ¹⁸F, can be used in positron emission tomography (PET) studies to determine substrate occupancy. The isotopically labeled compounds of the present disclosure can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., ²H or D) may afford certain therapeutic advantages resulting from greater metabolic stability (for example, increased *in vivo* half-life or reduced dosage requirements) and hence may be preferred in some circumstances in which deuterium substitution may be partial or complete; partial deuterium substitution refers to substitution of at least one hydrogen with at least one deuterium, and complete deuterium substitution refers to substitution of all hydrogens of the group with deuterium, for example, methyl (-CH₃) completely substituted with deuterium is -CD₃.

The compounds of the present disclosure may be present in their tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol and imine-enamine isomerizations. A specific example of a proton tautomer may be an imidazole moiety in which a proton can transfer between two ring nitrogens.

The pharmaceutical composition of the present disclosure can be prepared by combining the compound of the present disclosure with a suitable pharmaceutically acceptable excipient.

Typical routes of administration of the compound, the stereoisomer thereof, or the pharmaceutically acceptable salt thereof, or the pharmaceutical composition thereof of the present disclosure include, but are not limited to, oral, local, inhalation, parenteral, intranasal, intraocular, intramuscular, subcutaneous, and intravenous administration.

The pharmaceutical composition of the present disclosure can be manufactured using methods well known in the art, such as conventional mixing, dissolving, granulating, dragee-making, levigating, emulsifying, lyophilizing, etc. In all of the administration methods of the compound of formula (I) disclosed herein, the daily dose administered is from 0.001 mg/kg body weight to 2000 mg/kg body weight, given in individual or separated doses.

The compounds of the present disclosure can be prepared using a variety of synthetic methods well known to those skilled in the art, including the specific embodiments listed below, embodiments formed by combinations thereof with other chemical synthetic methods, and equivalent substitutions well known to those skilled in the art; preferred embodiments include, but are not limited to, the examples of the present disclosure.

The chemical reactions in the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthetic procedure or a reaction process based on the existing embodiments.

An important consideration in synthetic route planning in the art is the selection of suitable protecting groups for reactive functional groups (e.g., amino in the present disclosure); for example, reference may be made to Greene's Protective Groups in Organic Synthesis (4th Ed.) Hoboken, New Jersey: John Wiley & Sons, Inc, and all references cited herein are incorporated by reference in their entirety.

All patents, patent applications, and other identified publications are explicitly incorporated herein by reference for the purpose of description and disclosure. These publications are provided solely because they were disclosed prior to the filing date of the present disclosure. All statements as to the dates of these documents or descriptions as to the content of these documents are based on the information available to the applicant and do not constitute any admission as to the correctness of the dates or the content of these documents. Moreover, in any country or region, any reference to these publications herein shall not be construed as an admission that the publications form part of the commonly recognized knowledge in the art.

### The following abbreviations are used in the present disclosure:

PE represents petroleum ether; EA represents ethyl acetate; DCM represents dichloromethane; THF represents tetrahydrofuran; DMF represents N,N-dimethylformamide; TBDPSCl represents tert-butyldiphenylchlorosilane; DMAP represents 4-dimethylaminopyridine; dtbpy represents 4,4-di-tert-butylbipyridine; NIS represents N-iodosuccinimide; XantPhos represents 4,5-bis(diphenylphosphino)-9,9-dimethylxanthene; t-BuOK represents potassium tert-butoxide; Pd(dppf)Cl₂·CH₂Cl₂ represents [1,1'-bis(diphenylphosphino)ferrocene]palladium(II) dichloride dichloromethane complex; DIPEA represents N,N-diisopropylethylamine; HATU represents 2-(7-azabenzotriazol-1-yl)-N,N,N',N'-tetramethyluronium hexafluorophosphate; HOAT represents 1-hydroxy-7-azabenzotriazole; MeCN represents acetonitrile; Pd(dtbpf)Cl₂ represents [1,1'-bis(di-tert-butylphosphino)ferrocene]palladium(II) dichloride; EDCI represents 1-ethyl-3-(3-dimethylaminopropyl)carbodiimide hydrochloride; Boc represents tert-butoxycarbonyl; Cbz represents benzyloxycarbonyl; OAc represents acetoxy; TFA for trifluoroacetic acid.

### DETAILED DESCRIPTION

### Intermediate Example 1-A: Preparation of Intermediate A

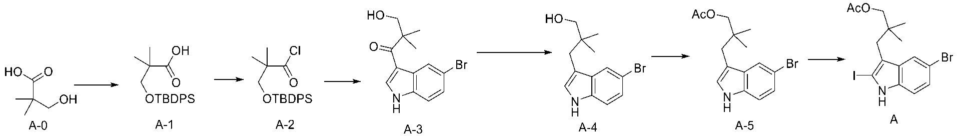

### Step 1:

Compound A-0 (50 g) and imidazole (32 g) were dissolved in dichloromethane (1 L). The solution was cooled to 0 °C, and TBDPSCl (120 mL) was added. After the dropwise addition was completed, the mixture was stirred at room temperature. After the reaction was completed, 2 N hydrochloric acid (1.2 L) was added to the reaction solution to quench the reaction, and the mixture was stirred and subjected to phase separation. The aqueous layer was extracted once with dichloromethane (1.2 L). The organic layers were combined, dried over anhydrous sodium sulfate, filtered, and concentrated to dryness by evaporation to give compound A-1 (154 g).

### Step 2:

Compound A-1 (154 g) was dissolved in toluene (500 mL), and thionyl chloride (40 mL) and DMF (1-3 drops) were added. The mixture was stirred at 80 °C. After the reaction was completed, the reaction solution was concentrated to dryness by evaporation under reduced pressure to give crude compound A-2 (162 g), which was directly used in the next step without purification.

### Step 3:

The crude compound A-2 (162 g) obtained in the previous step was dissolved in dichloromethane (360 mL), and the solution was cooled to 0 °C in an ice bath. A solution of anhydrous tin tetrachloride (117 g) in dichloromethane (400 mL) was slowly added dropwise. After the dropwise addition was completed, the mixture was stirred at 0 °C for another 0.5 h, and a solution of 5-bromoindole (75 g) in dichloromethane (120 mL) was added dropwise. The mixture was stirred in an ice-water bath for another 45 min and then stirred at room temperature. After the reaction was completed, the reaction solution was concentrated to dryness by evaporation under reduced pressure to remove the solvent. Ethyl acetate (500 mL) was added, and the mixture was stirred for complete dissolution and then washed three times with saturated brine (300 mL × 3). The aqueous layers were combined and extracted twice with ethyl acetate (200 mL × 2). The organic layers were combined, dried over anhydrous sodium sulfate, filtered, and concentrated to dryness by evaporation under reduced pressure. The crude product was slurried 3 times at room temperature with PE:EA = 2:1 (300 mL × 3), filtered under vacuum, and concentrated to dryness by evaporation to give compound A-3 (189 g).

### Step 4:

Compound A-3 (189 g) was dissolved in THF (372 mL), and the solution was stirred and cooled in an ice-water bath. Sodium borohydride (52.4 g) was added in 6 batches. After the addition was completed, the mixture was stirred at 60-65 °C. After the reaction was completed, methanol (200-300 mL) was added dropwise to the reaction solution to quench the reaction, and water (200 mL) was then added dropwise. The mixture was left to stand and filtered, and the filter cake was washed with EA (20 mL). The resulting filtrate was concentrated under reduced pressure. EA (500 mL) was added, and the mixture was stirred and filtered to remove insoluble substances. The organic layer was then washed twice with saturated brine (150 mL × 2), dried over anhydrous sodium sulfate, filtered, and concentrated to give compound A-4 (122 g).

### Step 5:

Compound A-4 (122 g) and triethylamine (179 mL) were dissolved in DCM (1 L). After the mixture was cooled in an ice-water bath for 1 h, acetic anhydride (66.1 g) was added. After 1 h of reaction, DMAP (4.1 g) was added. After 0.5 h of reaction, the ice bath was removed, and the system was stirred at room temperature. After the reaction was completed, water (200 mL) was added to the reaction solution, and the mixture was stirred and subjected to phase separation. The resulting aqueous layer was extracted twice with DCM (100 mL × 2). The organic layers were combined, washed three times with saturated brine (200 mL × 3), dried over anhydrous sodium sulfate, and filtered. The filtrate was concentrated, and the crude product was separated by silica gel column chromatography to give compound A-5 (69 g).

### Step 6:

Compound A-5 (10 g) was dissolved in THF (200 mL), and sodium bicarbonate (3.1 g), silver trifluoromethanesulfonate (9.5 g), and I₂ (7.8 g) were added in an ice bath. The mixture was stirred at 0 °C. After the reaction was completed, purified water (100 mL) was added to the reaction solution to quench the reaction, and the system was extracted twice with EA (300 mL × 2). The organic phases were combined, washed with a sodium sulfite solution (200 mL) and a sodium chloride solution (200 mL) in sequence, dried over anhydrous sodium sulfate, filtered under vacuum, concentrated, mixed with silica gel, and separated by silica gel column chromatography to give intermediate A (11.34 g). LC-MS: m/z: 449.9 (M-H)⁻.

### Intermediate Example 1-B: Preparation of Mixture of Intermediates B and B-4

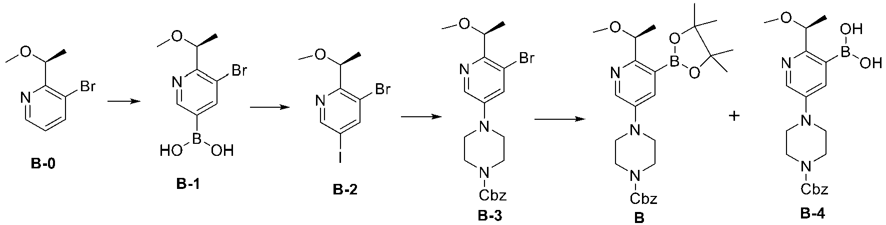

### Step 1:

Compound B-0 (80 g), bis(pinacolato)diboron (141 g), dtbpy (14.9 g), and (1,5-cyclooctadiene)(methoxy)iridium(I) dimer (7.5 g) were dissolved in THF (400 mL). The mixture was purged three times with nitrogen and stirred at 75 °C. After the reaction was completed, the reaction solution was cooled to room temperature, and sodium carbonate (40 g) and sodium hydroxide (10 g) were added to water (600 mL) for complete dissolution to give an aqueous base solution. Ethyl acetate (200 mL) was added to the reaction solution, and the pH was adjusted to 10 with the prepared aqueous base solution. The mixture was subjected to phase separation, and the aqueous phase was retained and extracted once with ethyl acetate (800 mL). The aqueous phase was cooled in an ice bath, and the pH was adjusted to 6 with a 6 N aqueous solution of diluted hydrochloric acid. A solid was precipitated and filtered under vacuum to give compound B-1 (62 g).

LC-MS: m/z: 260; 262.1 (M+H)⁺.

### Step 2:

Compound B-1 (16.3 g) and NIS (35.27 g) were added to acetonitrile (300 mL), and the mixture was stirred at 80 °C. After the reaction was completed, the reaction solution was cooled to room temperature. The reaction solution was washed twice with a saturated aqueous sodium thiosulfate solution (300 mL × 2) and extracted twice with EA (200 mL × 2). The organic phases were combined, washed with saturated brine, dried over anhydrous sodium sulfate, filtered, and concentrated. The crude product was separated by silica gel column chromatography (eluent: PE:EA = 20:1) to give compound B-2 (12 g).

LC-MS: m/z: 341.91; 343.92 (M+H)⁺.

### Step 3:

Compound B-2 (9.88 g), benzyl 1-piperazinecarbonate (15.73 g), XantPhos (1.68 g), Pd₂(dba)₃ (530.74 mg), and t-BuOK (4.88 g) were dissolved in toluene (200 mL). The solution was purged three times with argon and stirred at 120 °C. After the reaction was completed, the reaction solution was cooled to room temperature and concentrated under reduced pressure to remove most of the toluene. Water (150 mL) was added to the residue, and the mixture was extracted with EA (200 mL × 2). Washing was performed with saturated brine (200 mL × 2), and the organic phase was concentrated under reduced pressure. The crude product was separated by silica gel column chromatography (eluent: PE:EA = 1:1) to give compound B-3 (9.9 g).

LC-MS: m/z: 434.21; 436.19 (M+H)⁺.

### Step 4:

Compound B-3 (9.9 g), bis(pinacolato)diboron (8.68 g), potassium acetate (5.59 g), and Pd(dppf)Cl₂·CH₂Cl₂ (1.86 g) were dissolved in toluene (120 mL), and the solution was purged three times with argon and stirred at 90 °C. After the reaction was completed, the mixture was cooled to room temperature, concentrated under reduced pressure to remove most of the toluene, directly mixed with silica gel, and separated by column chromatography with neutral alumina to give a mixture of intermediates B and B-4 (13.6 g).

Intermediate B-4: LC-MS: m/z: 400.29 (M+H)⁺.

### Intermediate Example 1-C: Preparation of Intermediate C

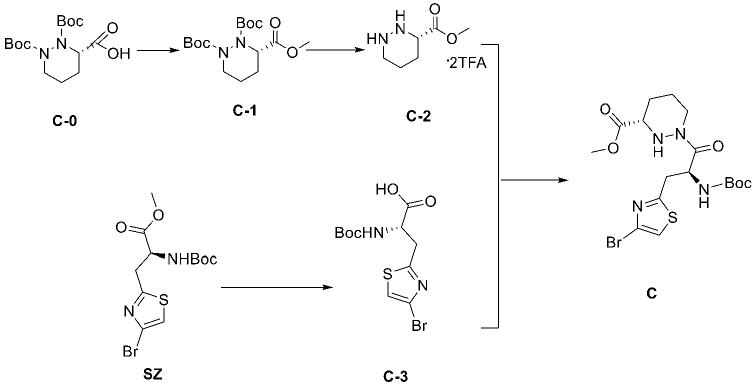

### Step 1:

Compound C-0 (27 g) and potassium carbonate (22.5 g) were dissolved in THF (400 mL). After stirring, a solution of iodomethane (12.7 g) in tetrahydrofuran (100 mL) was added dropwise. The mixture was stirred at room temperature. After the reaction was completed, the reaction solution was directly filtered, and the filter cake was washed. The filtrate was collected, concentrated, mixed with silica gel, and separated by silica gel column chromatography to give compound C-1 (23 g).

### Step 2:

Compound C-1 (23 g) was dissolved in dichloromethane (230 mL), and trifluoroacetic acid (115 mL) was added. The mixture was stirred at room temperature. After the reaction was completed, the reaction solution was concentrated to dryness by evaporation under reduced pressure to remove the solvent, and the residue was dried twice with toluene to give compound C-2 (25 g).

LC-MS: m/z: 145 (M+H)⁺.

### Step 3:

Compound SZ (10 g) and lithium hydroxide monohydrate (366.45 mg) were dissolved in tetrahydrofuran (50 mL) and water (25 mL), and the mixture was stirred at 40 °C. After the reaction was completed, THF was removed by concentration under reduced pressure, and DCM (100 mL) and water (50 mL) were added. The pH was adjusted to 5-6 with a 2 N aqueous solution of diluted hydrochloric acid, and extraction and phase separation were performed. The aqueous phase was extracted once with DCM (100 mL). The organic phases were combined, dried over anhydrous sodium sulfate, filtered, and concentrated to give compound C-3 (13.8 g).

### Step 4:

DIPEA (20 g) was added to a solution of compound C-2 (21.94 g) in dichloromethane (50 mL), and the resulting mixture was preserved for later use. Compound C-3 (13.8 g) was dissolved in DCM (200 mL), and the solution was cooled in an ice bath. The aforementioned mixture, HATU (19.33 g), HOAT (2.67 g), and DIPEA (30.79 g) were added, and the mixture was stirred at 0-5 °C. After the reaction was completed, water (500 mL) and DCM (200 mL) were added, and the mixture was stirred and subjected to phase separation. The organic phase was washed with 10% brine (500 mL), dried, filtered, and concentrated. The residue was separated by silica gel column chromatography (eluent: PE:EA = 2:1) to give intermediate C (16.2 g).

LC-MS: m/z: 477.1 (M+H)⁺.

### Intermediate Example 1-D: Preparation of Intermediate D

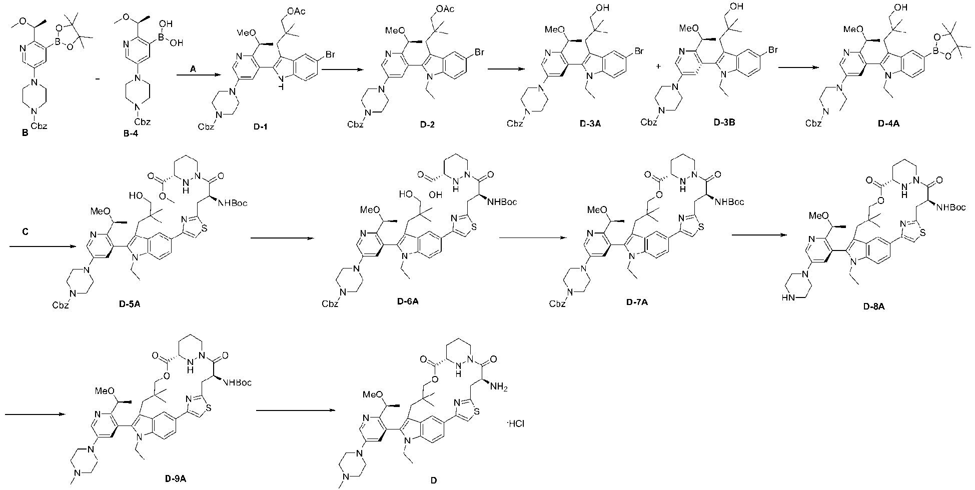

### Step 1:

The mixture of intermediates B and B-4 (8.38 g), intermediate A (6.3 g), K₂CO₃ (3.86 g), and 1,1'-bis(diphenylphosphino)ferrocenepalladium dichloride (2.53 g) were dissolved in a mixed solvent of toluene, 1,4-dioxane, and water (v/v/v = 3/1/1, 250 mL in total). The mixture was purged three times with nitrogen and stirred at 70 °C. After the reaction was completed, the reaction solution was extracted twice with water (150 mL) and ethyl acetate (150 mL × 2). The organic phase was washed once with saturated brine (150 mL), dried over anhydrous sodium sulfate, filtered, and concentrated. The residue was separated by silica gel column chromatography (eluent: PE:EA = 1:2) to give compound D-1 (5.28 g).

LC-MS: m/z: 677.2 (M+H)⁺.

### Step 2:

Compound D-1 (5.28 g) was added to DMF (50 mL), and the mixture was stirred for complete dissolution. Cesium carbonate (7.61 g) was added, and the mixture was cooled to 5 °C in an ice bath. Iodoethane (2.42 g) was added dropwise, and the mixture was stirred at 40 °C. After the reaction was completed, water (125 mL) and EA (150 mL) were added for extraction, and the organic phase was washed with 10% brine (150 mL), dried over anhydrous sodium sulfate, filtered, and concentrated to give crude D-2 (7 g).

LC-MS: m/z: 705.3 (M+H)⁺.

### Step 3:

The crude D-2 (7 g) obtained in the previous step was dissolved in methanol (50 mL), and LiOH (1.36 g) and K₃PO₄ (2.1 g) were added. The mixture was stirred at 40 °C. After the reaction was completed, the presence of atropisomers was monitored under liquid mass conditions (chromatographic column: Waters ACQUITY CSH C18; mobile phase A: 0.1% formic acid (FA)/H₂O; mobile phase B: MeCN; gradient elution (15%-95%-5%-15%) over 0-7 min), and the retention times of the prepeak and postpeak were 4.23 min and 4.40 min, respectively. The reaction solution was cooled to room temperature, directly mixed with silica gel, and separated by silica gel column chromatography (eluent: PE:EA = 1:2) to give two atropisomers of D-3, including prepeak compound D-3B (2.37 g) and postpeak compound D-3A (2.53 g).

LC-MS: m/z: 663.4 (M+H)⁺.

### Step 4:

Compound D-3A (2.5 g), bis(pinacolato)diboron (1.43 g), 1,1'-bis(diphenylphosphino)ferrocenepalladium dichloride (408.9 mg), and potassium acetate (736.96 mg) were dissolved in toluene (50 mL), and the solution was purged three times with nitrogen and stirred at 90 °C. After the reaction was completed, the reaction solution was cooled to room temperature, concentrated, mixed with silica gel, and separated by silica gel column chromatography (eluent: PE:EA = 1:1.5) to give compound D-4A (2.18 g).

LC-MS: m/z 711.5 (M+H)⁺.

### Step 5:

Compound D-4A (1.5 g), intermediate C (875.84 mg), Pd(dtbpf)Cl₂ (137.51 mg), and K₃PO₄ (1.11 g) were dissolved in 25 mL of a mixed solvent of toluene/dioxane/water (v/v/v = 3/1/1). The mixture was purged three times with nitrogen and stirred at 70 °C. After the reaction was completed, the reaction solution was cooled, concentrated, mixed with silica gel, and separated by silica gel column chromatography (eluent: PE:EA = 1:10) to give compound D-5A (1.67 g).

LC-MS: m/z 981.64 (M+H)⁺.

### Step 6:

Compound D-5A (1.25 g) and LiOH (175.72 mg) were dissolved in 17.5 mL of a mixed solvent of THF and H₂O (v/v = 2/1), and the mixture was stirred at 35 °C. After the reaction was completed, water (80 mL) and DCM (80 mL) were added to the system. The system was adjusted to pH 5-6 with a 2 N aqueous solution of diluted hydrochloric acid, extracted, and subjected to phase separation. The aqueous phase was extracted with DCM (80 mL). The organic phases were combined, dried over anhydrous sodium sulfate, filtered, and concentrated to give crude D-6A (1.3 g), which was used directly in the next step.

LC-MS: m/z 967.9 (M+H)⁺.

### Step 7:

The crude D-6A (1.3 g) was dissolved in DCM (150 mL), and EDCI (7.72 g), DIPEA (4.91 g), and HOBT (185 mg) were added. The mixture was stirred at room temperature. After the reaction was completed, water (300 mL) and DCM (200 mL) were added, and the mixture was extracted and washed. The organic phase was washed with water (300 mL) and saturated brine (200 mL × 2), dried over anhydrous sodium sulfate, and filtered, and the filtrate was concentrated. The crude product was subjected to silica gel column chromatography (eluent: DCM:MeOH = 50:1) to give compound D-7A (350 mg).

LC-MS: m/z 949.8 (M+H)⁺.

### Step 8:

Compound D-7A (350 mg) was dissolved in methanol (10 mL), and 10% Pd/C (100 mg) was added. The mixture was purged once with nitrogen, then purged 3 times with a hydrogen balloon, and stirred at 50 °C. After the reaction was completed, the reaction solution was cooled and filtered to remove Pd/C powder, and the filtrate was concentrated under reduced pressure to give crude D-8A, which was directly used in the next step.

LC-MS: m/z 815.4 (M+H)⁺.

### Step 9:

The crude D-8A obtained in the previous step was dissolved in methanol (15 mL), and 2 drops of acetic acid were added. The mixture was cooled in an ice bath. A 37% aqueous formaldehyde solution (107.76 mg) was added dropwise, and sodium cyanoborohydride (68.27 mg) was added. The mixture was stirred at room temperature. After the reaction was completed, the reaction solution was concentrated under reduced pressure to remove the solvent, and water (50 mL) and DCM (80 mL) were added for extraction. The organic phase was then washed with saturated brine (50 mL), dried over anhydrous sodium sulfate, and filtered, and the filtrate was concentrated to dryness under reduced pressure to give crude D-9A (336 mg).

LC-MS: m/z 829.4 (M+H)⁺.

### Step 10:

The crude D-9A (336 mg) obtained in the previous step was dissolved in 1,4-dioxane (5 mL), and the solution was cooled to 5 °C in an ice bath. A solution of hydrochloric acid in 1,4-dioxane (4 M, 2 mL) was added dropwise. After the dropwise addition was completed, the mixture was stirred at room temperature. After the reaction was completed, the reaction solution was concentrated by evaporation under reduced pressure to remove the solvent to give intermediate D (356 mg).

LC-MS: m/z 729.4 (M+H)⁺.

### Example 1: Preparation of Compound 1

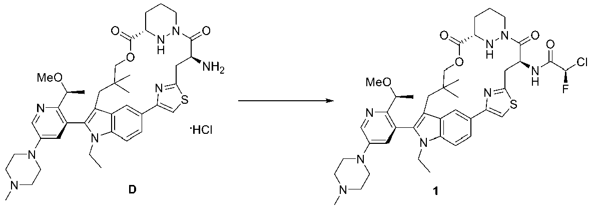

### Step 1:

Intermediate D (100 mg) was dissolved in DMF (10 mL), and (R)-2-chloro-2-fluoroacetic acid (18.51 mg), DIPEA (88.59 mg), and HATU (78.18 mg) were added. The mixture was stirred at room temperature. After the reaction was completed, the system was extracted twice with purified water (50 mL) and DCM (40 mL × 2). The organic phases were combined, washed with a saturated sodium chloride solution, dried over anhydrous sodium sulfate, filtered under vacuum, and concentrated. The crude product was subjected to preparative liquid phase chromatography (step 1: YMC AQ C18 chromatographic column; mobile phase A: 0.05% acetic acid/water; mobile phase B: acetonitrile; gradient: 10% B-50% B (0-60 min); step 2: mobile phase A: 20 mM ammonium acetate; mobile phase B: methanol; gradient: 30% B-90% B (0-60 min); step 3: mobile phase A: purified water; mobile phase B: acetonitrile; gradient: 10% B-10%-90%-90% B (0-20-21-60 min)) to give compound 1 (33 mg).

LC-MS: m/z 823.6 (M+H)⁺.

### Example 2: Preparation of Compound 2

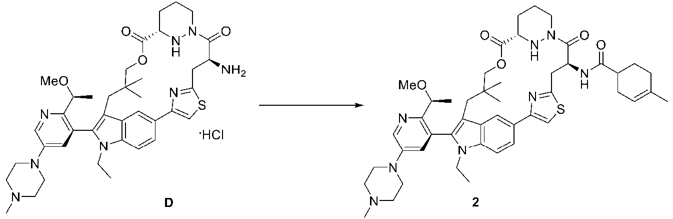

### Step 1:

Intermediate D (100 mg) was dissolved in DMF (10 mL), and 4-methyl-3-cyclohexene-1-carboxylic acid (23.06 mg), DIPEA (88.59 mg), and HATU (78.18 mg) were added. The mixture was stirred at room temperature. After the reaction was completed, the system was extracted twice with purified water (50 mL) and DCM (40 mL × 2). The organic phases were combined, washed with a saturated sodium chloride solution, dried over anhydrous sodium sulfate, filtered under vacuum, and concentrated. The crude product was subjected to preparative liquid phase chromatography (YMC AQ C18 chromatographic column, mobile phase A: a 0.05% aqueous acetic acid solution, mobile phase B: acetonitrile, gradient: 20% B-60% B, 0-60 min) to give compound 2 (65 mg).

LC-MS: m/z 851.5 (M+H)⁺.

### Example 3: Preparation of Compounds 3-A and 3-B

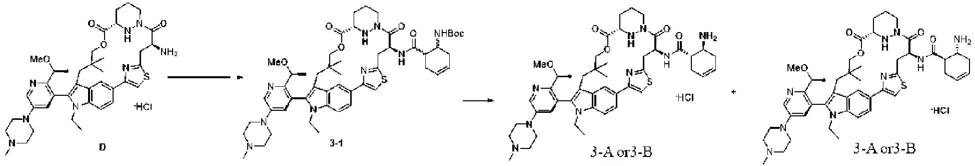

### Step 1:

Intermediate D (100 mg) was dissolved in DMF (10 mL), and Boc-(+/-)-trans-2-aminocyclohex-4-ene-1-carboxylic acid (CAS No. 865689-24-3) (39.69 mg), DIPEA (88.59 mg), and HATU (78.18 mg) were added. The mixture was stirred at room temperature. After the reaction was completed, purified water (50 mL) was added, and the mixture was extracted twice with DCM (40 mL × 2). The organic phases were combined, washed with a saturated sodium chloride solution, dried over anhydrous sodium sulfate, filtered under vacuum, and concentrated to give crude compound 3-1 (190 mg).

LC-MS: m/z 952.5 (M+H)⁺.

### Step 2:

The crude compound 3-1 (190 mg) obtained in the previous step was dissolved in 1,4-dioxane (5 mL), and a 4 M solution of hydrochloric acid in 1,4-dioxane (2 mL) was added. The mixture was stirred at room temperature. After the reaction was completed, the reaction solution was concentrated by evaporation under reduced pressure to remove the solvent, and the crude product was subjected to preparative liquid phase chromatography (YMC AQ C18 chromatographic column, mobile phase A: a 0.05% aqueous acetic acid solution, mobile phase B: acetonitrile, gradient: 10% B-40% B, 0-60 min) to give compound **3-B** (47 mg) with a retention time of 30 min and compound **3-A** (40 mg) with a retention time of 36 min in sequence.
Compound 3-A LC-MS: m/z: 852.51 (M+H)⁺;
Compound 3-B LC-MS: m/z: 852.48 (M+H)⁺.

### Examples 4-A and 4-B

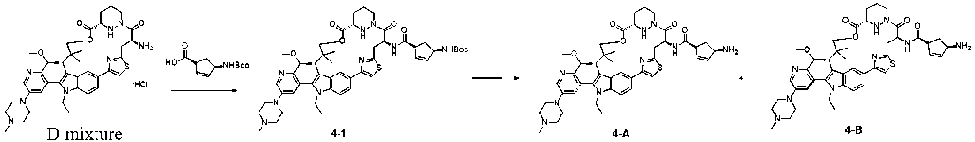

### Step 1:

Intermediate D (48 mg, containing a mixture of atropisomers) was dissolved in DMF (10 mL), and (1S,4R)-4-(Boc-amino)-2-cyclopentenic acid (17.03 mg), DIPEA (74.45 mg), and HATU (28.36 mg) were added. The mixture was stirred at room temperature. After the reaction was completed, purified water (30 mL) was added, and the mixture was extracted twice with EA (40 mL × 2), washed twice with brine, dried over anhydrous sodium sulfate for 2 h, and filtered. The filtrate was concentrated to dryness under reduced pressure to give crude compound 4-1 (68 mg).

### Step 2:

The crude compound 4-1 (68 mg) was added to 1,4-dioxane (5 mL), and a 4 M solution of HCl in 1,4-dioxane (1 mL) was added. The mixture was stirred at room temperature. After the reaction was completed, the reaction solution was concentrated by evaporation under reduced pressure to remove the solvent to give a crude product (82 mg). Purification was performed by preparative chromatography (chromatographic column: YMC AQ C18, 50 × 250 mm, 10 µm; mobile phase: A: 0.1% formic acid in water, B: methanol; gradient: 10%-80% B (0-60 min)). λ: 254 nm, V: 60 mL/min. Separation was performed to give a hydrochloride salt of compound 4-A (6 mg) at RT of 32.7 min and a hydrochloride salt of compound 4-B (14 mg) at RT of 38.6 min.

Compound 4-A LC-MS: m/z = 838.86 [M+H]⁺.

Compound 4-B LC-MS: m/z = 838.88 [M+H]⁺.

### Test Example 1: Assay for Inhibitory Activity for AsPc-1 Cell Proliferation

AsPc-1 cells in a well-growing state were collected into a centrifuge tube, adjusted to a cell density of 3 × 10⁴ cells/mL, and inoculated onto a 96-well plate (100 µL/well). The compounds were added using a nanoliter pipettor such that the final concentrations of the compounds were 5000 nM-0.31 nM (the addition was performed in duplicate). Meanwhile, a control was set. After 72 h of incubation in the cell incubator, the assay reagent CCK-8 (manufacturer: Dojindo Laboratories; 10 µL/well) was added. After 3 h of incubation in the cell incubator, the absorbance values were measured at 450 nm on a PerkinElmer Envision microplate reader. A four-parameter analysis was performed, a dose-response curve was fitted, and IC₅₀ was calculated. The experimental results are shown in Table 1. A represents that IC₅₀ < 100 nM; B represents that 100 nM ≤ IC₅₀ < 500 nM.

### Test Example 2: Assay for Inhibitory Activity for Capan-1 Cell Proliferation

Capan-1 cells in a well-growing state were collected into a centrifuge tube, adjusted to a cell density of 1.5 × 10⁴ cells/mL, and inoculated onto a 96-well plate (100 µL/well). The compounds were added using a nanoliter pipettor such that the final concentrations of the compounds were 20000 nM-9.1 nM (the addition was performed in duplicate). Meanwhile, a control was set. After 5 days of incubation in the cell incubator, the assay reagent CCK-8 (manufacturer: Dojindo Laboratories, Beijing; 10 µL/well) was added. After 4 h of incubation in the cell incubator, the absorbance values were measured at 450 nm on a PerkinElmer Envision microplate reader. A four-parameter analysis was performed, a dose-response curve was fitted, and IC₅₀ was calculated. The experimental results are shown in Table 1. A represents that IC₅₀ < 100 nM; B represents that 100 nM ≤ IC₅₀ < 500 nM.

**Table 1. Results of cell proliferation inhibition assay for compounds**

| Compound No. | Inhibitory activity IC₅₀ for AsPc-1 cell proliferation (nM) | Inhibitory activity IC₅₀ for Capan-1 cell proliferation (nM) |
|---|---|---|
| 1 | A | A |
| 2 | A | A |
| 3-A | B | A |
| 3-B | A | A |

The test results show that the compounds of the present disclosure exhibit relatively good inhibitory activity against the proliferation of AsPc-1 cells and Capan-1 cells.

### Test Example 3: In Vitro Liver Microsomal Stability

Liver microsome incubation samples (species: human and mouse) were each prepared by mixing a PBS buffer (pH = 7.4), a liver microsome solution (0.5 mg/mL), a test compound, and an NADPH + MgCl₂ solution, and incubated at 37 °C and 300 rpm for 1 h. Zero-hour samples were prepared by mixing a PBS buffer (pH = 7.4), a liver microsome solution (0.5 mg/mL), and a test compound. An acetonitrile solution containing an internal standard was added to the samples, and supernatants were prepared by protein precipitation, diluted, and then assayed by LC/MS/MS. The test results are shown in Table 2.

**Table 2. Results of in vitro liver microsomal metabolic stability**

| **Compound** | Remaining amount % (T = 60 min) (0.5 mg/mL) | |
|---|---|---|
| | Human liver microsome | Mouse liver microsome |
| 3-B | 33.03% | 80.9% |

The test results show that the compounds of the present disclosure exhibit *in vitro* liver microsomal metabolic stability.

## Claims

1. A compound of formula (I), a stereoisomer thereof, or a pharmaceutically acceptable salt thereof, 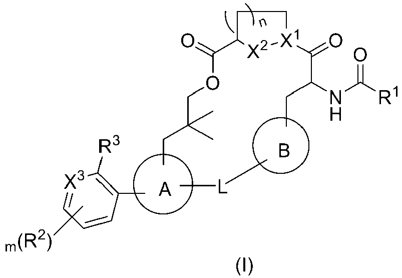 wherein
ring A is selected from the group consisting of C₆₋₁₀ aryl, 5- to 14-membered heteroaryl, and 5- to 14-membered heterocyclyl, wherein the C₆₋₁₀ aryl, 5- to 14-membered heteroaryl, or 5- to 14-membered heterocyclyl is optionally independently substituted with 1, 2, or 3 R^{a};
L is selected from the group consisting of -(CRR')_{q}-, -NH-(CRR')_{q}-, -O-(CRR')_{q}-, -S-(CRR')_{q}-, -(CRR')_{q}-(CH=CH)ᵢ-, -NH-(CH=CH)ᵢ-, -O-(CH=CH)ᵢ-, -S-(CH=CH)ᵢ-, -(CRR')_{q}-(C≡C)ᵢ-, -NH-(C≡C)ᵢ-, -O-(C≡C)ᵢ-, and -S-(C≡C)ᵢ-;
R and R' are each independently selected from the group consisting of hydrogen, deuterium, halogen, -NH₂, -OH, C₁₋₆ alkyl, C₁₋₆ haloalkyl, C₁₋₆ heteroalkyl, C₃₋₆ cycloalkyl, and 3- to 6-membered heterocyclyl, wherein the C₃₋₆ cycloalkyl or 3- to 6-membered heterocyclyl is optionally independently substituted with one or more substituents selected from the group consisting of halogen, =O, -NH₂, -OH, -SH, and -CN; or R and R', together with the carbon atom to which they are attached, form C=O, C₃₋₆ cycloalkyl, or 3- to 6-membered heterocyclyl, wherein the C₃₋₆ cycloalkyl or 3- to 6-membered heterocyclyl is optionally independently substituted with one or more substituents selected from the group consisting of halogen, =O, -NH₂, -OH, -SH, and -CN;
ring B is selected from the group consisting of C₃₋₁₄ cycloalkyl, C₃₋₁₄ cycloalkenyl, 3- to 14-membered heterocyclyl, C₆₋₁₀ aryl, and 5- to 14-membered heteroaryl, wherein the C₃₋₁₄ cycloalkyl, C₃₋₁₄ cycloalkenyl, 3- to 14-membered heterocyclyl, C₆₋₁₀ aryl, or 5- to 14-membered heteroaryl is optionally independently substituted with 1, 2, or 3 R^{b};
R^{a} and R^{b} are each independently selected from the group consisting of halogen, =O, -NH₂, -OH, -SH, -CN, C₁₋₁₂ alkyl, C₁₋₁₂ heteroalkyl, C₂₋₁₂ alkenyl, C₂₋₁₂ alkynyl, C₃₋₈ cycloalkyl, 3- to 8-membered heterocyclyl, phenyl, and 5- to 6-membered heteroaryl, wherein the C₁₋₁₂ alkyl, C₁₋₁₂ heteroalkyl, C₂₋₁₂ alkenyl, or C₂₋₁₂ alkynyl is optionally independently substituted with one or more substituents selected from the group consisting of halogen, -NH₂, -OH, -SH, and -CN; the C₃₋₈ cycloalkyl, 3- to 8-membered heterocyclyl, phenyl, or 5- to 6-membered heteroaryl is optionally independently substituted with one or more substituents selected from the group consisting of halogen, =O, -NH₂, -OH, -SH, -CN, -COOH, C₁₋₆ alkyl, C₁₋₆ heteroalkyl, hydroxyl-substituted C₁₋₆ alkyl, C₁₋₆ haloalkyl, and amino-substituted C₁₋₆ alkyl;
R¹ is selected from the group consisting of C₁₋₆ alkyl, C₁₋₆ heteroalkyl, C₂₋₆ alkenyl, C₂₋₆ alkynyl, C₃₋₁₄ cycloalkyl, C₄₋₁₄ cycloalkenyl, 4- to 14-membered heterocyclyl, C₆₋₁₀ aryl, and 5- to 14-membered heteroaryl, wherein the C₁₋₆ alkyl, C₁₋₆ heteroalkyl, C₂₋₆ alkenyl, or C₂₋₆ alkynyl is optionally independently substituted with one or more substituents selected from the group consisting of halogen, -NH₂, -OH, -SH, -CN, and -COOH; the C₃₋₁₄ cycloalkyl, C₄₋₁₄ cycloalkenyl, 4- to 14-membered heterocyclyl, C₆₋₁₀ aryl, or 5- to 14-membered heteroaryl is optionally independently substituted with one or more substituents selected from the group consisting of halogen, =O, -NH₂, -OH, -SH, -CN, -COOH, C₁₋₆ alkyl, C₁₋₆ heteroalkyl, hydroxyl-substituted C₁₋₆ alkyl, C₁₋₆ haloalkyl, and amino-substituted C₁₋₆ alkyl;
each R² is independently selected from the group consisting of halogen, -NH₂, -OH, -SH, -CN, -C(O)NHC₁₋₆ alkyl, -C(O)N(C₁₋₆ alkyl)₂, -C(O)OC₁₋₆ alkyl, -OC(O)C₁₋₆ alkyl, -N(C₁₋₆ alkyl)C(O)C₁₋₆ alkyl, -NHS(O)₂C₁₋₆ alkyl, -S(O)₂NHC₁₋₆ alkyl, -S(O)₂N(C₁₋₆ alkyl)₂, -P(O)(C₁₋₆ alkyl)₂, C₁₋₆ alkyl, C₁₋₆ heteroalkyl, C₂₋₆ alkenyl, C₂₋₆ alkynyl, C₃₋₁₄ cycloalkyl, C₄₋₁₄ cycloalkenyl, 4- to 14-membered heterocyclyl, C₆₋₁₀ aryl, and 5- to 14-membered heteroaryl, wherein the C₁₋₆ alkyl, C₁₋₆ heteroalkyl, C₂₋₆ alkenyl, or C₂₋₆ alkynyl is optionally independently substituted with one or more substituents selected from the group consisting of halogen, -NH₂, -OH, -SH, -CN, and -COOH; the C₃₋₁₄ cycloalkyl, C₄₋₁₄ cycloalkenyl, 4- to 14-membered heterocyclyl, C₆₋₁₀ aryl, or 5- to 14-membered heteroaryl is optionally independently substituted with one or more substituents selected from the group consisting of halogen, =O, -NH₂, -OH, -SH, -CN, -COOH, C₁₋₆ alkyl, C₁₋₆ heteroalkyl, hydroxyl-substituted C₁₋₆ alkyl, C₁₋₆ haloalkyl, and amino-substituted C₁₋₆ alkyl;
X¹ and X³ are each independently selected from the group consisting of CH and N;
X² is selected from the group consisting of CH₂ and NH;
n is selected from the group consisting of 1, 2, 3, and 4;
m is selected from the group consisting of 0, 1, 2, 3, and 4;
i is selected from the group consisting of 1 and 2;
q is selected from the group consisting of 0, 1, 2, and 3;
R³ is selected from the group consisting of hydrogen, halogen, -NH₂, -OH, -SH, -CN, C₁₋₆ alkyl, and C₁₋₆ heteroalkyl, wherein the C₁₋₆ alkyl or C₁₋₆ heteroalkyl is optionally independently substituted with 1, 2, or 3 substituents selected from the group consisting of halogen, -NH₂, -OH, -SH, and -CN.

2. The compound of formula (I), the stereoisomer thereof, or the pharmaceutically acceptable salt thereof according to claim 1, wherein ring A is selected from the group consisting of C₆₋₁₀ aryl, 5- to 10-membered heteroaryl, and 5- to 10- membered heterocyclyl, wherein the C₆₋₁₀ aryl, 5- to 10-membered heteroaryl, or 5- to 10-membered heterocyclyl is optionally independently substituted with 1, 2, or 3 R^{a};
or ring A is selected from the group consisting of C₆₋₁₀ aryl and 5- to 10-membered heteroaryl, wherein the C₆₋₁₀ aryl or 5- to 10-membered heteroaryl is optionally independently substituted with 1 or 2 R^{a};
or ring A is selected from the group consisting of phenyl, 5- to 6-membered heteroaryl, and 9- to 10-membered heteroaryl, wherein the phenyl, 5- to 6-membered heteroaryl, or 9- to 10-membered heteroaryl is optionally independently substituted with 1 or 2 R^{a};
or ring A is selected from the group consisting of phenyl and 5- to 6-membered or 9- to 10-membered heteroaryl, wherein the phenyl or 5- to 6-membered or 9- to 10-membered heteroaryl is optionally independently substituted with 1 or 2 substituents selected from the group consisting of halogen, C₁₋₄ alkyl, C₂₋₄ alkynyl, -NH₂, -OH, -SH, and -CN;
or ring A is selected from the group consisting of indolyl and pyrrolyl, wherein the indolyl or pyrrolyl is substituted with 1 or 2 ethyl or ethynyl.

3. The compound of formula (I), the stereoisomer thereof, or the pharmaceutically acceptable salt thereof according to claim 1 or 2, wherein L is selected from the group consisting of -(CRR')_{q}-, -(CRR')_{q}-(CH=CH)ᵢ-, and -(CRR')_{q}-(C≡C)ᵢ-;
or L is selected from the group consisting of -(CRR')_{q}- and -(CRR')_{q}-(CH=CH)ᵢ-;
or L is selected from the group consisting of a bond and -CH=CH-;
optionally, q is selected from the group consisting of 0, 1, and 2; or q is selected from the group consisting of 0 and 1; or q is selected from 0;
optionally, i is selected from 1.

4. The compound of formula (I), the stereoisomer thereof, or the pharmaceutically acceptable salt thereof according to any one of claims 1-3, wherein R and R' are each independently selected from the group consisting of hydrogen, deuterium, halogen, -NH₂, -OH, C₁₋₃ alkyl, C₁₋₃ haloalkyl, and C₁₋₃ heteroalkyl; or R and R', together with the carbon atom to which they are attached, form C=O or cyclopropyl;
or R and R' are each independently selected from the group consisting of hydrogen, F, Cl, -NH₂, -OH, and methyl;
or R and R' are each independently hydrogen.

5. The compound of formula (I), the stereoisomer thereof, or the pharmaceutically acceptable salt thereof according to any one of claims 1-4, wherein ring B is selected from the group consisting of C₅₋₁₀ cycloalkyl, C₅₋₁₀ cycloalkenyl, 5- to 10-membered heterocyclyl, C₆₋₁₀ aryl, and 5- to 10-membered heteroaryl, wherein the C₅₋₁₀ cycloalkyl, C₅₋₁₀ cycloalkenyl, 5- to 10-membered heterocyclyl, C₆₋₁₀ aryl, or 5- to 10-membered heteroaryl is optionally substituted with 1, 2, or 3 R^{b};
or ring B is selected from the group consisting of phenyl and 5- to 6-membered or 9- to 10-membered heteroaryl, wherein the phenyl or 5- to 6-membered or 9- to 10-membered heteroaryl is optionally independently substituted with 1 or 2 R^{b};
or ring B is selected from the group consisting of phenyl and 5- to 6-membered heteroaryl, wherein the phenyl or 5- to 6-membered heteroaryl is optionally independently substituted with 1 or 2 R^{b};
or ring B is selected from the group consisting of pyrrolyl, pyrazolyl, imidazolyl, oxazolyl, isoxazolyl, thiazolyl, isothiazolyl, triazolyl, furanyl, thienyl, pyridinyl, pyrazinyl, pyrimidinyl, and pyridazinyl, and ring B is optionally substituted with 1 or 2 R^{b};
or ring B is selected from the group consisting of phenyl and 5- to 6-membered heteroaryl, wherein the phenyl or 5- to 6-membered heteroaryl is optionally independently substituted with 1 or 2 substituents selected from the group consisting of -F, -Cl, -NH₂, -OH, -SH, and -CN;
or ring B is selected from the group consisting of phenyl and thiazolyl, wherein the phenyl is optionally substituted with 1 -OH;
or ring B is 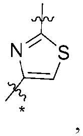 wherein * indicates that a bond at the position is linked to L.

6. The compound of formula (I), the stereoisomer thereof, or the pharmaceutically acceptable salt thereof according to any one of claims 1-5, wherein R^{a} and R^{b} are each independently selected from the group consisting of halogen, =O, -NH₂, -OH, -SH, -CN, C₁₋₈ alkyl, C₁₋₈ heteroalkyl, C₂₋₈ alkenyl, C₂₋₈ alkynyl, C₃₋₆ cycloalkyl, 3- to 6-membered heterocyclyl, phenyl, and 5- to 6-membered heteroaryl, wherein the C₁₋₈ alkyl, C₁₋₈ heteroalkyl, C₂₋₈ alkenyl, or C₂₋₈ alkynyl is optionally independently substituted with one or more substituents selected from the group consisting of halogen, -NH₂, -OH, -SH, and -CN; the C₃₋₆ cycloalkyl, 3- to 6-membered heterocyclyl, phenyl, or 5- to 6-membered heteroaryl is optionally independently substituted with one or more substituents selected from the group consisting of halogen, =O, -NH₂, -OH, -SH, -CN, -COOH, C₁₋₄ alkyl, C₁₋₄ heteroalkyl, hydroxyl-substituted C₁₋₄ alkyl, C₁₋₄ haloalkyl, and amino-substituted C₁₋₄ alkyl;
or optionally, R^{a} is selected from the group consisting of halogen, =O, -NH₂, -OH, -SH, -CN, C₁₋₃ alkyl, C₁₋₃ heteroalkyl, C₂₋₄ alkenyl, C₂₋₄ alkynyl, C₃₋₆ cycloalkyl, and 3- to 6-membered heterocyclyl, wherein the C₁₋₃ alkyl, C₁₋₃ heteroalkyl, C₂₋₄ alkenyl, or C₂₋₄ alkynyl is optionally independently substituted with one or more substituents selected from the group consisting of halogen, -NH₂, -OH, -SH, and -CN; the C₃₋₆ cycloalkyl or 3- to 6-membered heterocyclyl is optionally independently substituted with one or more substituents selected from the group consisting of halogen, =O, -NH₂, -OH, -SH, -CN, C₁₋₃ alkyl, C₁₋₃ heteroalkyl, and C₁₋₃ haloalkyl;
or R^{a} is selected from the group consisting of F, Cl, C₁₋₃ alkyl, -OC₁₋₃ alkyl, -NH(C₁₋₂ alkyl), -N(C₁₋₂ alkyl)₂, and C₂₋₄ alkynyl, wherein the C₁₋₃ alkyl, -OC₁₋₃ alkyl, -NH(C₁₋₂ alkyl), -N(C₁₋₂ alkyl)₂, or C₂₋₄ alkynyl is optionally independently substituted with one or more halogens;
or R^{a} is selected from the group consisting of ethyl and ethynyl;
or optionally, R^{b} is selected from the group consisting of halogen, =O, -NH₂, -OH, -SH, -CN, C₁₋₆ alkyl, C₁₋₆ heteroalkyl, C₂₋₆ alkenyl, and C₂₋₆ alkynyl, wherein the C₁₋₆ alkyl, C₁₋₆ heteroalkyl, C₂₋₆ alkenyl, or C₂₋₆ alkynyl is optionally independently substituted with one or more substituents selected from the group consisting of halogen, -NH₂, -OH, -SH, and -CN;
or R^{b} is selected from the group consisting of halogen, -NH₂, -OH, -SH, -CN, and C₁₋₃ alkyl.

7. The compound of formula (I), the stereoisomer thereof, or the pharmaceutically acceptable salt thereof according to any one of claims 1-6, wherein R¹ is selected from the group consisting of C₁₋₆ alkyl, C₁₋₆ heteroalkyl, C₂₋₆ alkenyl, C₂₋₆ alkynyl, C₃₋₁₀ cycloalkyl, C₅₋₁₀ cycloalkenyl, 4- to 10-membered heterocyclyl, C₆₋₁₀ aryl, and 5-to 10-membered heteroaryl, wherein the C₁₋₆ alkyl, C₁₋₆ heteroalkyl, C₂₋₆ alkenyl, or C₂₋₆ alkynyl is optionally independently substituted with one or more substituents selected from the group consisting of halogen, -NH₂, -OH, -SH, -CN, and -COOH; the C₃₋₁₀ cycloalkyl, C₅₋₁₀ cycloalkenyl, 4- to 10-membered heterocyclyl, C₆₋₁₀ aryl, or 5-to 10-membered heteroaryl is optionally independently substituted with one or more substituents selected from the group consisting of halogen, =O, -NH₂, -OH, -SH, -CN, -COOH, C₁₋₆ alkyl, C₁₋₆ heteroalkyl, and C₁₋₆ haloalkyl; or R¹ is selected from C₃₋₆ cycloalkyl, and n is 1, wherein the C₃₋₆ cycloalkyl is optionally substituted with one or more substituents selected from the group consisting of halogen, =O, -NH₂, -OH, -SH, -CN, C₁₋₃ alkyl, -OC₁₋₃ alkyl, C₁₋₃ haloalkyl, -NH(CH₃), and -N(CH₃)₂;
or R¹ is selected from C₃₋₆ cycloalkyl, and ring A is not 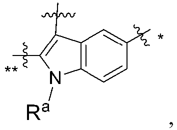 wherein the C₃₋₆ cycloalkyl is optionally substituted with one or more substituents selected from the group consisting of halogen, =O, -NH₂, -OH, -SH, -CN, C₁₋₃ alkyl, -OC₁₋₃ alkyl, C₁₋₃ haloalkyl, -NH(CH₃), and -N(CH₃)₂, wherein * indicates that a bond at the position is linked to L, and ** indicates that a bond at the position is linked to the structural fragment 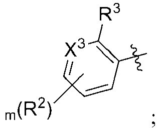
or R¹ is selected from the group consisting of C₅₋₈ cycloalkenyl and 5- to 8-membered partially unsaturated heterocyclyl, wherein the C₅₋₈ cycloalkenyl or 5- to 8-membered partially unsaturated heterocyclyl is optionally independently substituted with one or more substituents selected from the group consisting of halogen, =O, -NH₂, -OH, -SH, -CN, C₁₋₃ alkyl, -OC₁₋₃ alkyl, C₁₋₃ haloalkyl, -NH(CH₃), and -N(CH₃)₂;
or R¹ is selected from the group consisting of cyclohexenyl, cyclopentenyl, and bicyclo[2.2.1]hept-2-enyl, wherein the cyclohexenyl, cyclopentenyl, or bicyclo[2.2.1]hept-2-enyl is optionally independently substituted with 1 or 2 F, Cl, -NH₂, CHF₂, or methyl;
or R¹ is selected from the group consisting of C₁₋₃ haloalkyl and C₅₋₇ cycloalkenyl, wherein the C₅₋₇ cycloalkenyl is optionally substituted with 1 or 2 substituents selected from the group consisting of halogen, -NH₂, C₁₋₃ alkyl, and C₁₋₃ haloalkyl;
or R¹ is selected from the group consisting of halomethyl, 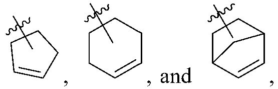 wherein the 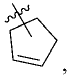 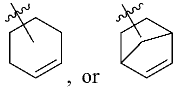 is optionally independently substituted with 1 or 2 substituents selected from the group consisting of halogen, -NH₂, C₁₋₃ alkyl, and C₁₋₃ haloalkyl;
or R¹ is selected from the group consisting of 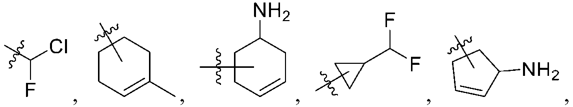 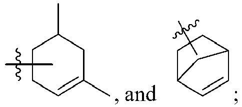
or R¹ is selected from the group consisting of

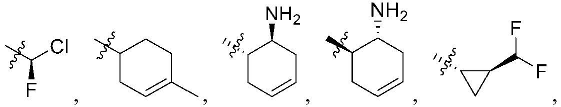

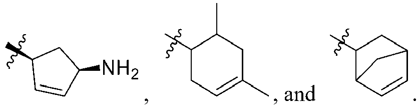

8. The compound of formula (I), the stereoisomer thereof, or the pharmaceutically acceptable salt thereof according to any one of claims 1-7, wherein each R² is independently selected from the group consisting of halogen, -NH₂, -OH, -SH, -CN, -C(O)NH(C₁₋₃ alkyl), -C(O)N(C₁₋₃ alkyl)₂, -C(O)OC₁₋₃ alkyl, -OC(O)C₁₋₃ alkyl, -N(C₁₋₃ alkyl)C(O)(C₁₋₃ alkyl), -NHS(O)₂(C₁₋₃ alkyl), -S(O)₂NH(C₁₋₃ alkyl), -S(O)₂N(C₁₋₃ alkyl)₂, -P(O)(C₁₋₃ alkyl)₂, C₁₋₆ alkyl, C₁₋₆ heteroalkyl, C₂₋₆ alkenyl, C₂₋₆ alkynyl, C₃₋₁₀ cycloalkyl, C₃₋₁₀ cycloalkenyl, 3- to 10-membered heterocyclyl, C₆₋₁₀ aryl, and 5- to 10-membered heteroaryl, wherein the C₁₋₆ alkyl, C₁₋₆ heteroalkyl, C₂₋₆ alkenyl, or C₂₋₆ alkynyl is optionally independently substituted with one or more substituents selected from the group consisting of halogen, -NH₂, -OH, -SH, -CN, and -COOH; the C₃₋₁₀ cycloalkyl, C₃₋₁₀ cycloalkenyl, 3- to 10-membered heterocyclyl, C₆₋₁₀ aryl, or 5- to 10-membered heteroaryl is optionally independently substituted with one or more substituents selected from the group consisting of halogen, =O, -NH₂, -OH, -SH, -CN, C₁₋₄ alkyl, C₁₋₄ heteroalkyl, and C₁₋₄ haloalkyl;
or each R² is independently selected from the group consisting of C₃₋₈ cycloalkyl, C₅₋₈ cycloalkenyl, and 5- to 8-membered heterocyclyl, wherein the C₃₋₈ cycloalkyl, C₅₋₈ cycloalkenyl, or 5- to 8-membered heterocyclyl is optionally independently substituted with one or more halogens, -NH₂, -OH, -SH, -CN, C₁₋₃ alkyl, -OC₁₋₃ alkyl, C₁₋₃ haloalkyl, -NH(CH₃), or -N(CH₃)₂;
or each R² is independently selected from 5- to 6-membered heterocyclyl containing 1 or 2 N or O atoms, wherein the 5- to 6-membered heterocyclyl is optionally substituted with one or more substituents selected from the group consisting of halogen, C₁₋₃ alkyl, and C₁₋₃ haloalkyl;
or each R² is independently selected from 5- to 8-membered heterocycloalkyl, wherein the 5- to 8-membered heterocycloalkyl is optionally substituted with one or more substituents selected from the group consisting of halogen, C₁₋₃ alkyl, and C₁₋₃ haloalkyl;
or each R² is independently selected from 5- to 6-membered heterocycloalkyl containing 1 or 2 N atoms, wherein the 5- to 6-membered heterocycloalkyl is optionally substituted with 1 or 2 substituents selected from the group consisting of F, Cl, and C₁₋₃ alkyl;
or each R² is independently selected from piperazinyl, wherein the piperazinyl is optionally substituted with methyl;
or each R² is independently selected from 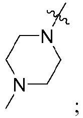
optionally, m is selected from the group consisting of 0, 1, and 2;
or m is selected from the group consisting of 0 and 1; or m is selected from 0; or m is selected from 1.

9. The compound of formula (I), the stereoisomer thereof, or the pharmaceutically acceptable salt thereof according to any one of claims 1-8, wherein X¹ is selected from N, X² is selected from the group consisting of CH₂ and NH, and X³ is selected from the group consisting of CH and N;
or X¹ and X³ are selected from N, and X² is selected from the group consisting of CH₂ and NH;
or X¹ and X³ are selected from N, and X² is selected from CH₂;
or X¹ and X³ are selected from N, and X² is selected from NH;
optionally, n is selected from the group consisting of 1, 2, and 3;
or n is selected from the group consisting of 1 and 2;
or n is selected from 1; or n is selected from 2.

10. The compound of formula (I), the stereoisomer thereof, or the pharmaceutically acceptable salt thereof according to any one of claims 1-9, wherein R³ is selected from the group consisting of hydrogen, halogen, -NH₂, -OH, -SH, -CN, C₁₋₄ alkyl, and C₁₋₄ heteroalkyl, wherein the C₁₋₄ alkyl or C₁₋₄ heteroalkyl is optionally independently substituted with 1, 2, or 3 substituents selected from the group consisting of halogen, -NH₂, -OH, -SH, and -CN;
or R³ is selected from C₁₋₃ heteroalkyl;
or R³ is selected from the group consisting of F, Cl, -NH₂, -OH, -CN, C₁₋₃ alkyl, and C₁₋₃ heteroalkyl, wherein the C₁₋₃ alkyl or C₁₋₃ heteroalkyl is optionally independently substituted with 1 or 2 F atoms;
or R³ is selected from

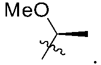

11. The compound of formula (I), the stereoisomer thereof, or the pharmaceutically acceptable salt thereof according to any one of claims 1-10, wherein the compound of formula (I), the stereoisomer thereof, or the pharmaceutically acceptable salt thereof is selected from the group consisting of a compound of formula (I-1), a compound of formula (I-1A), a compound of formula (I-2), a compound of formula (I-2A), a compound of formula (I-3), a compound of formula (I-3A), a compound of formula (I-4), a compound of formula (I-4A), a compound of formula (I-5), and a compound of formula (I-5A) below, and stereoisomers thereof and pharmaceutically acceptable salts thereof: 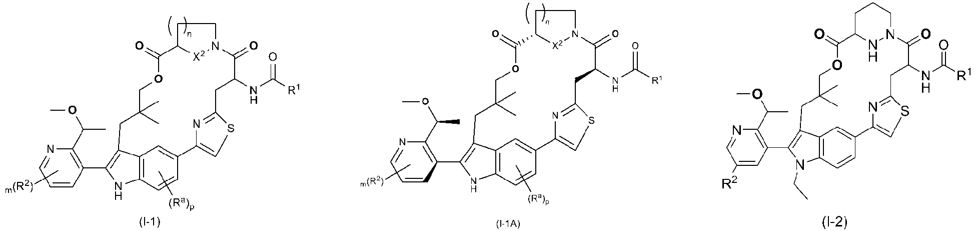 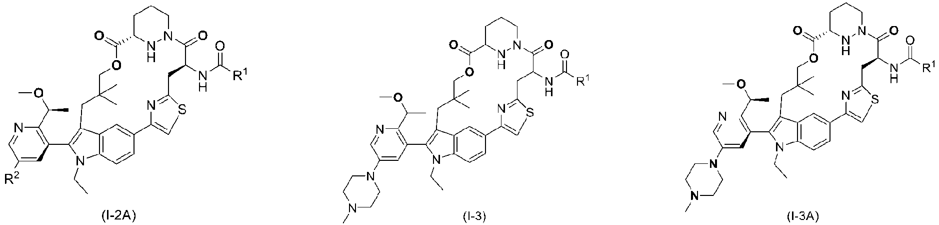 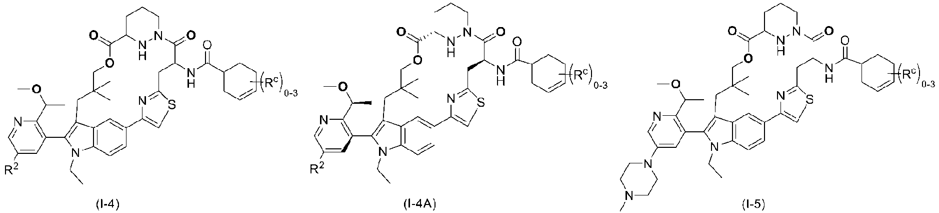 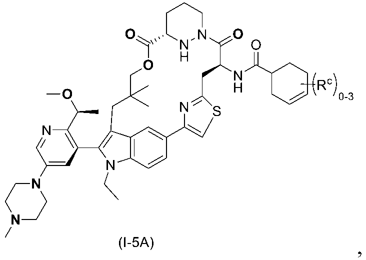 wherein p is selected from the group consisting of 1, 2, and 3; each R^{c} is independently selected from the group consisting of halogen, =O, -NH₂, -OH, -SH, -CN, -COOH, C₁₋₆ alkyl, C₁₋₆ heteroalkyl, hydroxyl-substituted C₁₋₆ alkyl, C₁₋₆ haloalkyl, and amino-substituted C₁₋₆ alkyl.

12. Compounds as follows, stereoisomers thereof, or pharmaceutically acceptable salts thereof:

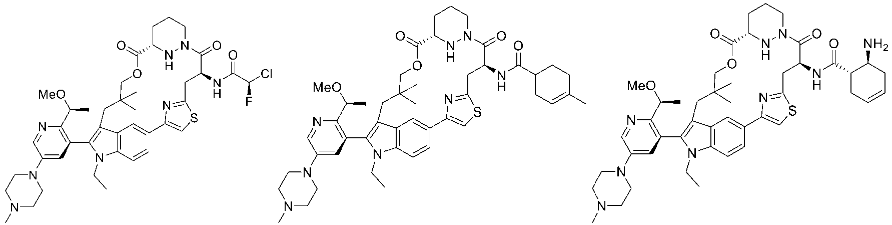

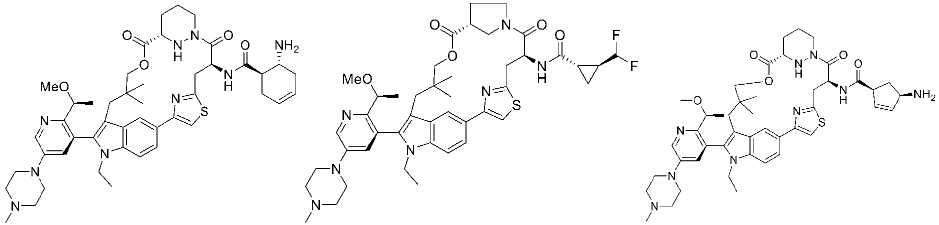

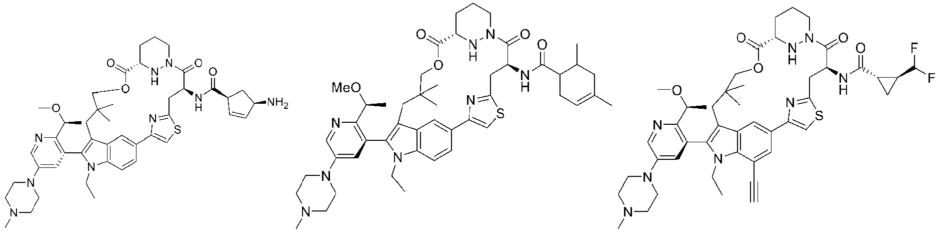

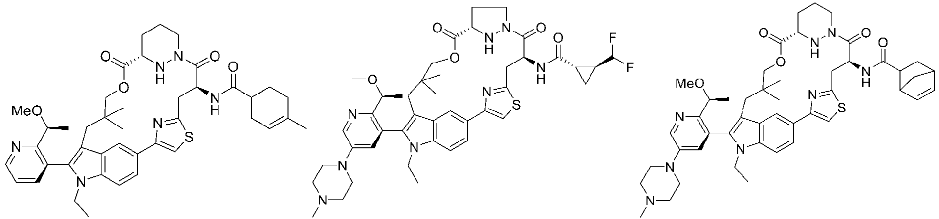

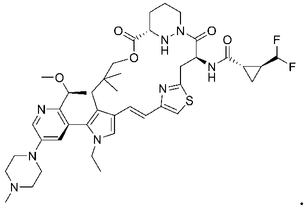

13. A pharmaceutical composition, comprising the compound, the stereoisomer thereof, or the pharmaceutically acceptable salt thereof according to any one of claims 1-12.

14. Use of the compound, the stereoisomer thereof, or the pharmaceutically acceptable salt thereof according to any one of claims 1-12, or the pharmaceutical composition according to claim 13 for preparing a medicament for treating a Ras protein-related disease, wherein optionally, the Ras protein-related disease is selected from cancer.
